(19) Europäisches Patentamt
European Patent Office
Office européen des brevets

(11) Publication number: **0 208 431**
**B1**

(12) **EUROPEAN PATENT SPECIFICATION**

(45) Date of publication of patent specification: **11.07.90**

(21) Application number: **86304521.7**

(22) Date of filing: **12.06.86**

(51) Int. Cl.$^5$: **A 01 N 43/58**, A 01 N 47/14, A 01 N 55/00, A 01 N 55/02, C 07 F 7/10, C 07 D 237/10, C 07 D 237/12

(54) **Fungicidal pyridazines.**

(30) Priority: **14.06.85 US 744722**

(43) Date of publication of application:
**14.01.87 Bulletin 87/03**

(45) Publication of the grant of the patent:
**11.07.90 Bulletin 90/28**

(84) Designated Contracting States:
**AT BE CH DE FR GB IT LI LU NL SE**

(56) References cited:
**DE-A-2 531 476**
**DE-A-3 014 991**
**DE-A-3 139 569**
**GB-A-1 090 950**
**US-A-3 883 530**
**US-A-4 263 297**

(73) Proprietor: **ELI LILLY AND COMPANY**
**Lilly Corporate Center**
**Indianapolis Indiana 46285 (US)**

(72) Inventor: **Dow, William Clinton**
**2710 Gamble Court**
**Hayward California 94542 (US)**
Inventor: **Johnson, George William**
**603 Waterview Boulevard**
**Greenfield Indiana 46140 (US)**
Inventor: **Arnold, Wendell Ray**
**11803 Eden Glen Drive**
**Carmel Indiana 46032 (US)**

(74) Representative: **Hudson, Christopher Mark et al**
**Erl Wood Manor**
**Windlesham Surrey GU20 6PH (GB)**

**EP 0 208 431 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention belongs to the fields of agricultural and organic chemistry, and provides to the art a series of new 4-(halo-branched-alkyl or silyl)-pyridazines, which are fungicides for the protection of plants from harmful pathogens. The compounds are effective against Phycomycetous fungi, which include some of the most injurious plant pathogens. In particular, the compounds are effective against *Phytophthora infestans*, which is the causative organism of late blight of potato and tomato.

Halo-branched-alkyl heterocycles have not previously been studied in the plant protection art. Earlier work has focused on compounds such as those of U.S. Patent 3,883,530, which teaches di- and trichloromethylpyridazines having from 2 to 3 chlorine atoms on the heterocyclic ring. U.S. Patent 4,263,297 shows a 3-trichloromethylpyridazine having an alkoxy group at the 6-position. Both patents describe their compounds as fungicides and bactericides.

The present invention provides a series of pyridazines of the formula (I):

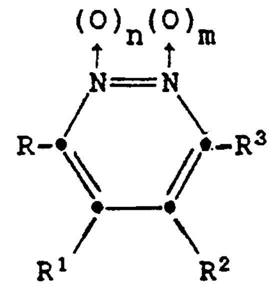

(I)

wherein

$R^3$ is chloro, bromo, methyl, cyano or iodo;

R is chloro, bromo, iodo, methyl, cyano or furan-2-ylmethoxy;

$R^1$ is hydrogen, methyl, ethyl or *n*-propyl;

$R^2$ is

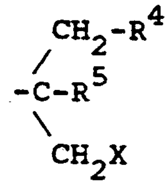

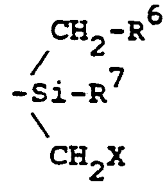

or

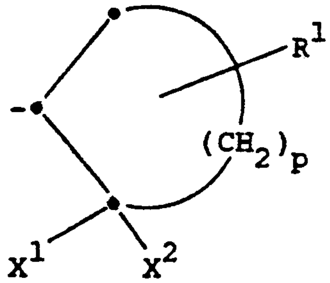

X is fluoro, chloro, bromo or iodo;

$X^1$ and $X^2$ independently represent X or hydrogen, provided that no more than one of $X^1$ and $X^2$ is hydrogen;

$R^4$ is hydrogen, chloro, bromo, methyl or ethyl;

$R^5$ is hydrogen, chloro, methyl, ethyl, chloromethyl or dichloromethyl;

or $R^4$ and $R^5$ combine with the group to which they are attached to form a $C_3$—$C_7$ cycloalkyl group substituted with a $R^1$ group;

$R^6$ is hydrogen, chloro, bromo, methyl or ethyl;
$R^7$ is hydrogen, methyl, ethyl, chloromethyl or dichloromethyl;
one of m and n is 0 or 1, and the other is 0;
p is 0—4.

The invention also provides a process for preparing the compounds of the formula (I) which comprises:

a) halogenating a compound of Formula I, wherein $R^2$ is defined as

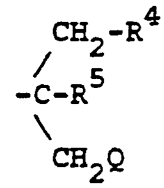

or

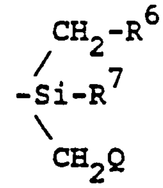

or

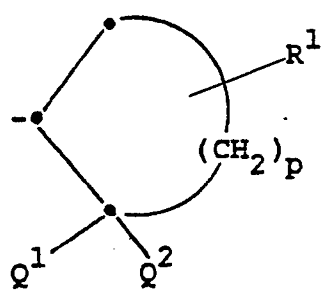

wherein Q or one or both of $Q^1$ and $Q^2$ are hydroxy, hydrogen or a suitable leaving group; or
b) displacement of one or more of Q, $Q^1$ and $Q^2$ halogen atoms with a different halogen;
c) halogenating a compound of Formula I, wherein either one or both of R and $R^3$ are hydroxy,
d) displacing an $R^1$ or $R^3$ halo group, with a different halogen,
e) N-oxidation of a compound of Formula I wherein n and/or m are 0, or
f) displacement of an R or $R^3$ halo group with furan-2-ylmethanol.

The invention also provides a method of reducing the adverse effects of Phycomycetous fungi on plants which comprises applying a Phycomycete-inhibiting amount of a compound of the invention to the plant or to the soil in which it grows.

The invention also provides fungicidal compositions which comprise a compound of the invention and a phytologically-acceptable carrier.

The invention further provides fungicidal combination compositions and methods comprising a compound of the invention in combination with a dithiocarbamate fungicide of the formula

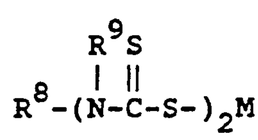

wherein
$R^8$ is $C_1$—$C_4$ alkylene;
$R^9$ is $C_1$—$C_3$ alkyl or hydrogen;
M is a divalent metal ion or two monovalent metal ions;
or

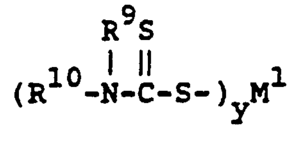

wherein

y is 1—3;

$M^1$ is a metal ion of valence 1—3;

$R^{10}$ is $C_1$—$C_4$ alkyl.

Throughout this document, all temperatures will be described in degrees Celsius. All expressions of percentage, proportion and the like will be in weight units unless otherwise stated.

In the structural formula above, all of the chemical terms carry their conventional meanings.

While the above structural formula is believed to describe the compounds unambiguously, a group of the exemplary compounds will be mentioned to assure that the reader fully understands the invention.

3,6-dichloro-4-(1-bromomethylethyl)pyridazine
3,6-dichloro-4-(1-chloromethyl-1-methylethyl)-5-propylpyridazine
3,6-dichloro-4-(1-fluoromethylpropyl)-5-propylpyridazine
4-(1-bromomethyl-1-chloromethylpropyl)-3-chloro-6-(furan-2-ylmethoxy)pyridazine
3,6-dichloro-4-(1-chloromethyl-1-iodomethylbutyl)-5-methylpyridazine
3-chloro-4-(1-dichloromethyl-1-fluoromethylethyl)-6-(furan-2-ylmethoxy)pyridazine
3-chloro-4-(1-chloromethylethyl)-6-(furan-2-ylmethoxy)pyridazine
4-(1-bromomethyl-1-ethylbutyl)-3,6-dichloro-5-ethylpyridazine
3,6-dichloro-4-(1-chloromethyl-1-iodomethylpropyl)pyridazine
3,6-dichloro-4-(1-bromomethyl-1-chloromethylethyl)-5-methylpyridazine, $N^2$-oxide
4-(1-bromomethyl-1-methylpropyl)-3-chloro-6-(furan-2-ylmethoxy)pyridazine, $N^1$-oxide
3-bromo-4-(1-bromomethyl-1-chloro-2-chloroethyl)-6-methylpyridazine
4-(2-bromo-1-chloro-1-methylethyl)-3-chloro-6-cyanopyridazine, $N^2$-oxide
4-(1-bromomethyl-1-fluoromethylpropyl)-5-ethyl-3,6-diiodopyridazine
4-(1-chloro-1-iodomethylbutyl)-3,6-dimethylpyridazine
3,6-dibromo-4-(1-chloromethylcyclopropyl)-5-methylpyridazine
4-(1-bromomethyl-2-methylcyclobutyl)-3,6-dicyanopyridazine
3-bromo-6-cyano-4-(4-ethyl-1-fluoromethylcyclohexyl)pyridazine
6-cyano-3-iodo-4-(1-iodomethyl-3-propylcycloheptyl)-5-propylpyridazine
4-(chloromethyl)(dichloromethyl)methylsilyl-6-(furan-2-ylmethoxy)-3-methylpyridazine
4-(chloromethyl)(iodomethyl)ethylsilyl-3-cyano-5-ethyl-6-(furan-2-ylmethoxy)pyridazine, $N^1$-oxide
4-(bromomethyl)(chloromethyl)fluoromethylsilyl-6-(furan-2-ylmethoxy)-3-iodopyridazine
3-bromo-4-(bromomethyl)(chloromethyl)ethylsilyl-6-(furan-2-ylmethoxy)pyridazine
4-(bromomethyl)(methyl)ethylsilyl-3,6-dichloropyridazine
3-bromo-4-(chloromethyl)(iodomethyl)silyl-6-cyanopyridazine
6-bromo-4-(chloromethyl)(dichloromethyl)methylsilyl-3-methylpyridazine, $N^2$-oxide
4-(fluoromethyl)methylsilyl-6-iodo-3-methylpyridazine
6-bromo-4-(bromomethyl)(chloromethyl)ethylsilyl-3-chloropyridazine
6-bromo-4-(bromomethyl)(methyl)ethylsilyl-3-chloropyridazine
3,6-dichloro-4-(2-fluorocyclopropyl)pyridazine
4-(2-bromocyclopentyl)-3,6-diiodopyridazine, $N^1$-oxide
4-(2-iodo-3-methylcyclobutyl)-3,6-dimethylpyridazine
4-(2-chlorocycloheptyl)-6-(furan-2-ylmethoxy)-3-methylpyridazine
3-bromo-4-(2-ethyl-6-iodocyclohexyl)-6-methylpyridazine
6-bromo-4-(2-chloro-4-propylcyclopentyl)-3-methylpyridazine
6-bromo-4-(2-fluorocyclopropyl)-3-iodopyridazine
3-bromo-4-(2-bromo-5-methylcyclohexyl)-6-iodopyridazine
3,6-dichloro-4-(2-chlorocycloheptyl)pyridazine
3-bromo-4-(2-chlorocyclobutyl)-6-cyanopyridazine

While all of the compounds of the present invention are useful and desirable for fungicidal purposes, certain classes of the compounds are preferred.

The following formula shows one preferred class of compounds.

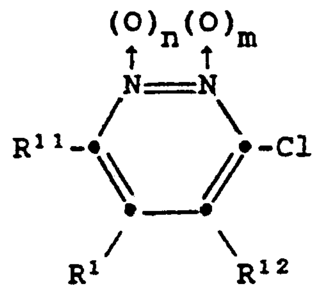

wherein

$R^{11}$ is chloro or furan-2-ylmethoxy;

$R^{12}$ is

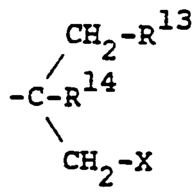

$R^{13}$ is hydrogen, chloro, methyl or ethyl;
$R^{14}$ is hydrogen, methyl, ethyl, chloromethyl or dichloromethyl;
$R^1$, X, m and n are defined above.

The following group of limitations describe other preferred classes of the compounds. It will be understood that the limitations described below can be combined to create further groups of preferred compounds.

a) R is chloro, bromo or methyl;
b) R is chloro or bromo;
c) R is chloro;
d) $R^3$ is chloro, bromo or methyl;
e) $R^3$ is chloro or bromo;
f) $R^3$ is chloro;
g) $R^3$ is furan-2-ylmethoxy;
h) $R^1$ is hydrogen;
i) $R^2$ is

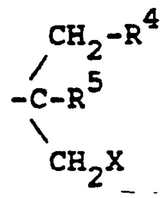

j) $R^2$ is

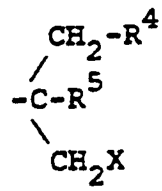

or

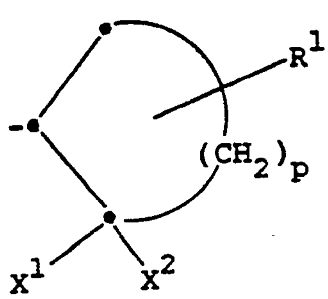

k) X is bromo or chloro;
l) X is fluoro or iodo;
m) $R^4$ is hydrogen, chloro or bromo;
n) $R^4$ is hydrogen or chloro;
o) $R^4$ is methyl or ethyl;
p) $R^4$ is hydrogen;
q) $R^5$ is hydrogen, chloro or methyl;
r) $R^5$ is hydrogen, methyl or ethyl;
s) $R^5$ is chloromethyl or dichloromethyl;

t) $R^5$ is methyl;

u) $R^5$ is hydrogen or ethyl;

v) m and n are 0.

The key intermediate for the preparation of the compounds is the pyridazine which has the R and $R^3$ groups in place. The 3,6-dichloropyridazine is a commercial product. Other halogenated intermediates can be prepared in manners analogous to the preparation of the dichloro compound. For example, the dibromopyridazine is most conveniently prepared by reacting 3,6-dioxopyridazine with phosphorus oxybromide. The other halogenated pyridazines are prepared analogously.

Intermediates having one halogen at the 3-position and another at the 6-position can be prepared by displacing a bromine or chlorine atom with a halogenating reagent, conveniently, with a lithium halide.

Intermediates having one methyl group are prepared, for example, by reacting levulinic acid with hydrazine to prepare 3-methyl-6-oxopyridazine, which compound can be halogenated, as with phosphorus oxychloride, to prepare a 3-methyl-6-halo intermediate. Intermediates having a methyl at both the 3- and 6-positions are most conveniently prepared from 2,5-dimethylfuran, which is reacted with methanol in the presence of bromine to give the 2,5-dimethyl-2,5-dimethoxy compound. That intermediate is reacted with hydrazine to give the desired 3,6-dimethyl intermediate.

Intermediate pyridazines having a cyano group are conveniently prepared from the corresponding carboxylic acids, by converting the acid to an amide, and dehydrating the amide to form the desired cyano group.

The group $R^2$ is put in place on the 3,6-disubstituted pyridazine by processes which are referred to, in general, as alkylation. The alkylation processes will be discussed with reference to the preferred group

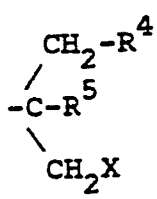

Alkylations to put other $R^2$ groups in place will then be more briefly discussed.

The preferred first step is an alkylation with a propanediol of the formula

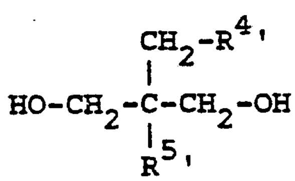

A

wherein $R^{4'}$ and $R^{5'}$ represent the non-halogenated definitions of the groups $R^4$ and $R^5$. For example, if the group $R^5$ is to be chloromethyl in the product, the group $R^{5'}$ will be methyl in formula A.

The starting compound of formula A is used to alkylate the pyridazine in an alkylation of the type which has been described in many articles by Minisci and co-workers, see for instance, *Synthesis*, *1*, 1—24 (1973). In general, the reaction goes in the presence of silver ion and persulfate ion ($S_2O_8^{--}$) in an aqueous acid, preferably sulfuric acid or trifluoroacetic acid. The process is carried out at moderate temperatures, in the range of from about the ambient temperature to about 100°, and the alkylations produce economically useful yields in moderate periods of time in the range of from several minutes to several hours. It is advisable to use a substantial excess of the propanediol alkylating agent, and a substantial excess of the persulfate as well. An amount of silver ion in the range of from about a few tenths of a mole, to about one mole per mole of product to be obtained is effective. It is preferred to use a relatively large amount of silver ion and recover the silver.

The alkylation conditions remove one of the hydroxymethyl groups from the propanediol, in the form of formaldehyde, and produce a product wherein the 4-position of the pyridazine is occupied by a group of the formula

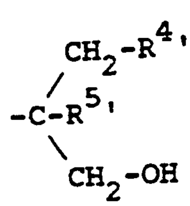

B

Alternatively, the alkylation is performed with a hydroxypropanaldehyde of Formula $A_1$

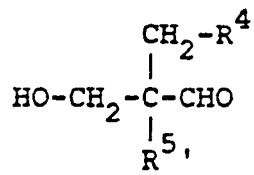

$A_1$

Use of the aldehyde has the advantage that the alkylation can be done without the presence of silver ion. In other respects, alkylations with the aldehyde are carried out under the same conditions as alkylations with the propanediol, and the same group of formula B is provided at the 4-position of the pyridazine.

The hydroxy group of the group of formula B is replaced by a halogen to provide the group X of the compounds of this invention. Conventional halogenating agents are used. When X is to be chloro, the preferred agent is thionyl chloride, used in the presence of pyridine under anhydrous conditions. Temperatures in the range of about 50—100° give economically-acceptable yields of the chlorinated product in several hours. Example 1 below illustrates the process.

Another convenient way to provide the halogen atom X is first to esterify the compound having the group of formula B with an acid, of which *p*-toluenesulfonic acid is particularly convenient. Reaction of the compound with, for example, *p*-toluenesulfonyl chloride, usually in the presence of an acid scavenger, provides the desired ester. That compound can be halogenated with simple halides, such as lithium bromide, potassium iodide, lithium chloride and the like, to provide the desired compound wherein X is the corresponding halogen atom.

As is well known, suitable fluorinating agents are few, because of the great stability of fluorine compounds. One compound which can be used to replace the hydroxy group of the group of formula B with fluorine is diethylaminosulfur trifluoride. The reaction is carried out under basic conditions, in the presence of a strong organic base such as triethylamine, pyridine and the like, in a highly stable solvent, such as halogenated alkane. Dichloromethane, chloroform and the like are suitable. The reaction should be carried out under anhydrous conditions and at low temperatures, in the range of about −25° to 25°.

When the groups $R^4$ and $R^5$ include halogen atoms, the compound is halogenated by conventional means. It is preferred, in most cases, to carry out such halogenations after the halogen atom X has been inserted, unless all halogens in the group are the same.

The halogenations of the groups $R^{4'}$ and $R^{5'}$ are carried out under free radical conditions, usually in the presence of activating energy such as strong light, and preferably in the presence of a radical initiator. Convenient halogenating agents for use under such conditions include, for example, sulfuryl chloride, N-iodosuccinimide, N-bromosuccinimide, N-chlorosuccinimide, and the like. Organic peroxides are the preferred initiators, of which benzoyl peroxide is particularly useful. Other initiators, for example, azo-bisisobutyronitrile, *t*-butyl hydroperoxide and the like, can also be used if desired. Only a catalytic amount of the initiator is needed. The processes are carried out in highly inert solvents, of which halogenated alkanes such as carbon tetrachloride are preferred. Such reactions are carried out at moderate temperatures, in the range of from about the ambient temperature to about 100°, and are often most effectively carried out continuously in equipment which flows the reaction mixture in a thin film past the strong light which provides activating energy.

If it is desired to avoid the use of a propanediol or propanaldehyde intermediate, the pyridazine intermediate can be alkylated with the alkanoic acid which corresponds to the $R^2$ group without the halogen atoms in place. For example, if $R^2$ is to be 1-chloromethyl-1-methylethyl, the alkanoic acid would be pivalic acid. The alkylation is carried out under Minisci conditions, substantially as discussed above for alkylations with a propanediol. When such a starting compound is used, the group X must be placed by a halogenation under free radical conditions, as described above, as are other chlorine atoms in the groups $R^3$ and $R^4$.

When the $R^2$ group is an alkylsilyl group, it is put in place by a free radical technique. The halogen atoms of the silyl $R^2$ group can be in place on the starting compound. The process is conveniently carried out in the presence of a radical initiator, such as was described above, under high-energy conditions such as strong light. It is very important to carry out the silylation under perfectly dry conditions. The silyl intermediate may have a hydrogen atom as the fourth bond to the silicon atom, or may have a halogen.

When the $R^2$ group is one wherein $R^4$ and $R^5$ combine to form a cycloalkyl group, it is most conveniently prepared by starting with a 1,1-di(hydroxymethyl)cycloalkyl intermediate. The alkylation is carried out under Minisci conditions as described above to prepare a 1-hydroxymethylcycloalkyl group. The hydroxymethyl group is then halogenated as has been discussed in detail.

The preparation of halocycloalkyl groups conveniently begins with a cycloalkene intermediate. The alkylation is carried out under Minisci conditions to prepare the compound having a 2-hydroxycycloalkyl substituent at the 4-position. The hydroxy group is replaced with a halogen atom in the same way that other halogenations are carried out.

When the desired product has a 5-alkyl substituent, that substituent is preferably inserted as a later step, by an alkylation with the corresponding alkanoic acid under Minisci conditions as described above.

For example, if a 5-methyl group is desired, the compound is alkylated with acetic acid; if a propyl group is desired, it is alkylated with butyric acid.

When a product is desired wherein R is furan-2-ylmethoxy, the 6-chlorine atom is replaced by a simple reaction with furan-2-ylmethanol, carried out in the presence of an acid scavenger such as a strong inorganic or organic base. Strong bases such as alkali metal hydrides and hydroxides, alkyllithium compounds, and dialkylamides, especially butyllithium and diisopropyl amide, are particularly useful. The process is carried out under anhydrous conditions in the presence of a solvent which is inert to the strong base. Amides such as dimethylformamide and dimethylacetamide are particularly useful.

The N-oxides of the present invention are easily prepared in the usual way, by simple oxidation of the pyridazine. Usually an oxidation is carried out as the last step in the synthetic procedure. The usual oxidizing agents for such processes are organic peroxy acids, of which peroxybenzoic acid and the chloroperoxybenzoic acids are typical. Oxidations are carried out readily near the ambient temperature, for example, at the reflux temperature of a reaction mixture in a halogenated alkane solvent such as dichloromethane and the like.

In general, it is advisable to use excess amounts of the relatively inexpensive reactants in the above process steps, to assure that the more expensive or harder to obtain reactants are fully utilized. As the examples below illustrate, excess amounts in the range of from about 10% to about 100%, or even up to several hundred percent, can beneficially be used when the economics of the process justify doing so.

The following preparative examples further illustrate the synthesis of the present compounds, and assure that the reader can obtain any desired compound.

Preparation 1

3,6-dichloro-4-(1-hydroxymethyl-1-methylethyl)pyridazine

To a 5-liter flask were added 341 g of 2,2-dimethyl-1,3-propanediol, 500 ml of water, 223 g of 3,6-dichloropyridazine, 100 ml of sulfuric acid in 900 ml of water and 51 g of silver nitrate. To the mixture was then added, dropwise, with an insulating mantle around the flask, 600 g of ammonium persulfate dissolved in 1 liter of water. The addition was carried out in about 20 minutes, while the temperature rose from 33° to 86°. When the addition was complete, the insulating mantle was removed and the flask was placed in a water bath to cool it. When the temperature had reached 40°, 1200 ml of dichloromethane was added and the mixture was stirred for 10 minutes more and filtered through a polypropylene filter pad. The solids were washed with 500 ml of dichloromethane, and the layers of the combined filtrate were separated. The aqueous layer was extracted with 1 liter of dichloromethane, and the combined organic layers were extracted with 1 liter of water, and dried over sodium sulfate. The solvent was removed under vacuum, to leave 385 g of a gummy solid. Most of the solid was removed and dissolved in 1200 ml of toluene at 85°. It was then cooled to 0° and filtered, and the solids were washed with cold toluene and dried under vacuum to obtain 152 g of the desired intermediate product, 97% pure by nuclear magnetic resonance analysis, m.p. 133—136°.

Preparation 1A

3,6-dichloro-4-(1-hydroxymethyl-1-methylethyl)-pyridazine

Ten ml of water and 0.33 ml of sulfuric acid were heated to 80°, and to it were added 0.75 g of 3,6-dichloropyridazine and 2.55 g of 3-hydroxy-2,2-dimethylpropanaldehyde. Then a solution of 5.7 g of ammonium persulfate in 15 ml of water was added dropwise over 10 minutes. The temperature of the mixture reached 100° during the addition. The mixture was stirred for one hour, and then 0.51 g of the aldehyde and 1.1 g of ammonium persulfate were added, and the mixture was stirred one hour more at 90°. It was then cooled and extracted twice with 30 ml portions of dichloromethane. The organic layers were combined, washed with 30 ml of water and then with 30 ml of saturated aqueous sodium bicarbonate, and dried with sodium sulfate. The solvent was removed under vacuum to obtain 1.38 g of oil, which was chromatographed on 100 g of silica gel, eluting with one liter of 3:7 ethyl acetate:hexane and then with 2:3 ethyl acetate:hexane. The product-containing fractions were combined and evaporated under vacuum to obtain 0.43 g of the desired product in crude form, about 80% pure by nuclear magnetic resonance analysis. The spectrum, taken in $CDCl_3$ on a 90 mHz instrument, showed the following characteristic features: δ 7.59 (s, 1H); 4.02 (broad s, 3H); 1.46 (s, 6H).

Preparation 2

3,6-dichloro-4-(1-hydroxymethyl-1-methylethyl)pyridazine

To a 500 ml flask were added 22.2 g of 2,2-dimethyl-1,3-propanediol, 100 ml of water, 14.9 g of 3,6-dichloropyridazine and 9.2 ml of trifluoroacetic acid. The mixture was heated to 37° and 17.0 g of silver nitrate was added. To the mixture was then added 39.9 g of ammonium persulfate, dissolved in 75 ml of water, over 8 minutes. The temperature increased as soon as the addition began, and ultimately reached 80°. The mixture was then cooled to ambient temperature, and 100 ml of dichloromethane was added. It was stirred for 5 minutes and filtered, and the solids were washed with 50 ml of dichloromethane, which was added to the filtrate. The layers of the filtrate were separated, and the aqueous layer was washed with 100 ml of dichloromethane. The organic layers were combined and washed twice with 100 ml portions of water. The organic layer was then dried with sodium sulfate and evaporated under vacuum. The tacky residue was

dissolved in 50 ml of toluene at 95° and filtered while still hot. The solution was cooled to 0° and filtered, and the solids were washed with cold toluene and vacuum dried to obtain 13.0 g of the desired intermediate. The filtrate was concentrated under vacuum and was chromatographed on silica gel with 40% ethyl acetate in hexane to obtain 3.6 g of additional product.

The silver salts which had been recovered in the first filtration step were washed with methanol and vacuum dried to obtain 10.6 g of silver salts. The aqueous layer from the first filtrate was treated with ammonium chloride, and the resulting precipitate was filtered and dried to obtain 2.3 g of additional silver salt.

Example 1

3,6-dichloro-4-(1-chloromethyl-1-methylethyl)pyridazine

A 140 g portion of the above intermediate was slurried in 660 ml of dry toluene, and 61 ml of dry pyridine was added. To the mixture was added, over a period of 7 minutes, 55.5 ml of thionyl chloride. The temperature increased to 55°, and a condenser was placed on the vessel and the mixture was heated to and held at 75° for 10 hours. The mixture was then allowed to cool, with stirring, over 6 hours. An additional 6.1 ml portion of pyridine and 5.5 ml of thionyl chloride were added, and the mixture was heated again to 75° and stirred at that temperature for 14 hours. It was then cooled, and 300 ml of 1N hydrochloric acid was added. The mixture warmed to 45°, and was stirred until it reached ambient temperature again. The organic layer was separated, and was washed with two 300 ml portions of water and then with 300 ml of brine. It was then dried over sodium sulfate and evaporated under vacuum to a thick oil. The oil crystallized upon cooling, and was dried further without heating to remove remaining toluene. It was then recrystallized by adding it to 300 ml of boiling isopropanol, and cooling the solution to ambient temperature over 1 hour, with scratching. The mixture was filtered, and the filter cake was washed twice with 50 ml portions of cold isopropanol and was vacuum dried to obtain 108.6 g of the desired product, m.p. 66.5—68.5°. Further concentration and crystallization of the isopropanol produced 13.4 g of additional product. The combined product was analyzed by high performance liquid chromatography against an authentic standard, with 3:2 methanol:water as the eluant on a Zorbax ODS column, using ultraviolet detection at 254 nm. The product was indicated to be 100% pure. Its elemental analysis was as follows.

Theory: C, 40.11; H, 3.79; N, 11.43;
Found: C, 39.88; H, 3.52; N, 11.43.

Example 2

3,6-dichloro-4-(1-bromomethyl-1-methylethyl)pyridazine

A 22.1 g portion of the product of Preparation 1 was dissolved in 65 ml of pyridine, and was added over a period of 15 minutes to a slurry of 21 g of *p*-toluenesulfonyl chloride in 10 ml of pyridine. The temperature dropped to 18° at first, and then rose to 30°. The mixture was stirred at ambient temperature for 16 hours, and then 100 ml of dichloromethane was added and the mixture was cooled to 0°. To it was added 70 ml of concentrated hydrochloric acid, while the temperature was held below 30°. The layers were separated, and the aqueous layer was extracted with 100 ml of dichloromethane. The organic layers were combined and washed twice with 100 ml portions of water and then with 100 ml of brine. The organic layer was then dried with magnesium sulfate, and was evaporated to dryness under vacuum to obtain 34.9 g of a solid. Five g of the solids were removed, and the rest was recrystallized from 120 ml of boiling isopropanol to obtain 26.3 g of white 3,6-dichloro-4-(1-*p*-toluenesulfonyloxymethyl-1-methylethyl)pyridazine, m.p. 110—113°.

A 37.5 g portion of the above intermediate, obtained by successive reactions, was combined with 13.9 g of lithium bromide in 100 ml of dry dimethylsulfoxide under nitrogen, and the mixture was heated to 110° and stirred at that temperature for 2 hours. The mixture was then cooled to ambient temperature, and 100 ml of water was added dropwise. When one-half of the water had been added, 10 mg of crystals of the desired product was added, as seeds. When all of the water was in, the mixture was stirred until the temperature fell to 25°, and an additional 15 minutes. The mixture was then filtered, and the filter cake was washed three times with 50 ml portions of water, and was vacuum dried to obtain 27.7 g of solid. It was recrystallized by adding it to 55 ml of boiling isopropanol and cooling to 0°. The mixture was filtered and the solids were washed with cold isopropanol to obtain 25.9 g of dry product, m.p. 86.5—88.5°. Its elemental analysis was as follows.

Theory: C, 33.84; H, 3.19; N, 9.86; Cl, 24.97; Br, 28.14;
Found: C, 33.89; H, 3.10; N, 9.80; Cl, 25.43; Br, 29.12.

Example 3

3,6-dichloro-4-(1-fluoromethyl-1-methylethyl)pyridazine

Fifteen g of the product of Preparation 1 was dissolved in 450 ml of dichloromethane and 13.8 g of triethylamine, and the solution was cooled to 5° under nitrogen. To it was added 16.5 g of diethylaminosulfur trifluoride, in small portions. A mild exotherm resulted but the temperature did not rise above 10°. The mixture was then stirred at 5° for 3.5 hours, and was slowly added to 400 ml of cold water. Sodium bicarbonate was added until the aqueous layer was neutral, and the organic layer was then separated and washed with 200 ml of 1N hydrochloric acid and 100 ml of brine. It was then dried over magnesium sulfate, and evaporated under vacuum to an oil. The oil was dissolved in 50 ml of

dichloromethane, and the solution was filtered through 50 ml of silica gel. The filtrate was evaporated under vacuum to a pasty solid, which was purified by high performance liquid chromatography, eluting with 9:1 heptane:ethyl acetate. The product-containing fractions were combined and evaporated under vacuum, and the residue was re-chromatographed in the same manner to obtain about 70 mg of the desired product, m.p. 36—38°. The product's identity was confirmed by nuclear magnetic resonance analysis on a 60-mHz instrument in $CDCl_3$, which revealed the following features: δ 1.53 (d, 6H, J=2Hz, *gem*-$CH_3$); 4.65 (d, 2H, J=47Hz, —$CH_2F$); 7.43 (s, 1H, aromatic).

Preparation 3

3,6-dichloro-4-(1-ethyl-1-hydroxymethylpropyl)pyridazine

Twenty g of 3,6-dichloropyridazine, 2.3 g of silver nitrate and 44.4 g of 2,2-diethyl-1,3-propanediol were slurried in 280 ml of water and 19.7 g of sulfuric acid. To the mixture were added, simultaneously, solutions of 61.3 g of ammonium persulfate in 200 ml of water and 20 g of silver nitrate in 40 ml of water, over a period of 20 minutes at 55°. The mixture heated exothermically to 70°, and it was cooled to ambient temperature after the addition was complete. The aqueous layer was then decanted off, and the solids remaining were slurried twice in 800 ml of diethyl ether. The resulting solution was shaken with the aqueous layer, and the layers were separated again. The aqueous layer was then made basic to pH 9 with ammonium hydroxide, and was re-extracted with 500 ml of diethyl ether. The ether was evaporated under vacuum to obtain 46 g of brown oil, which was purified by high performance liquid chromatography, eluting with 6:1 heptane:ethyl acetate. The product-containing fractions were combined and evaporated under vacuum to obtain, after recrystallization from diethyl ether/heptane, 0.54 g of the desired product, m.p. 85—87°.

Example 4

3,6-dichloro-4-(1-chloromethyl-1-ethylpropyl)pyridazine

A 1.3 g portion of the product of Preparation 3 was collected by concentrating the impure fractions from the chromatography above, mixed with an equal amount of a byproduct, 4-chloro-6,6-diethylpyridazino[3,4-b]furan. It was combined with 1.9 g of thionyl chloride and 1.2 g of pyridine in 40 ml of toluene, and the mixture was stirred at 73° for 18 hours. An additional 1.2 g of pyridine and 1.9 g of thionyl chloride were added, and the mixture was stirred at 73° for 2.5 hours more. It was then evaporated under vacuum to a semi-solid, and was dissolved in 150 ml of diethyl ether and 70 ml of 1N hydrochloric acid. The organic layer was separated, and was washed with brine and dried over magnesium sulfate. It was then evaporated under vacuum to obtain 1.2 g of oil, which was chromatographed over 100 ml of silica gel, eluting with 6:1 heptane:ethyl acetate. The product-containing fractions were combined, and the impure product was re-chromatographed over 50 ml of silica gel, eluting with 9:1 heptane:ethyl acetate to obtain 270 mg of the desired product, m.p. 45—47°. Its elemental analysis was as follows.

Theory: C, 44.89; H, 4.90; N, 10.47;
Found: C, 44.86; H, 4.69; N, 10.48.

Preparation 4

3,6-dichloro-4-(1-hydroxymethyl-1-methylbutyl)pyridazine

Twenty g of 3,6-dichloropyridazine was alkylated with 44.4 g of 2-methyl-2-propyl-1,3-propanediol, following the process of Preparation 3 above. In this case the chromatography was run with 3:1 heptane:ethyl acetate as the eluant, and the residue from evaporation of the combined product-containing fractions was recrystallized from diethyl ether/heptane to obtain 0.43 g of the desired intermediate product, m.p. 99—100°.

Example 5

3,6-dichloro-4-(1-chloromethyl-1-methylbutyl)pyridazine

One g of the product of Preparation 4, obtained from successive reactions, was dissolved in 25 ml of toluene and 0.95 g of pyridine. To the solution was added 1.43 g of thionyl chloride dissolved in 10 ml of toluene, dropwise, over 10 minutes at ambient temperature. The mixture was then stirred at 75° for 20 hours, and was then cooled and evaporated under vacuum. The pasty residue was dissolved in 150 ml of diethyl ether and 70 ml of water, and the organic layer was separated. It was washed with 50 ml of water, and with 50 ml of brine, and was dried over magnesium sulfate and evaporated under vacuum to obtain 1.2 g of oil. It was chromatographed over 50 ml of silica gel, eluting with 9:1 heptane:ethyl acetate. The product-containing fractions were combined and evaporated to obtain 0.42 g of the desired product, m.p. 68—69°. Its elemental analysis was as follows.

Theory: C, 44.89; H, 4.90; N, 10.47;
Found: C, 45.10; H, 4.66; N, 10.45.

Preparation 5

3,6-dichloro-4-isopropylpyridazine

Fifty g of 3,6-dichloropyridazine was slurried in 500 ml of water with 28.5 g of silver nitrate and 66.5 g of isobutyric acid at 50°. Five hundred ml of water and 98.6 g of sulfuric acid were added, and the mixture was

heated to 60°. To it was added 228 g of ammonium persulfate, dissolved in 500 ml of water. The mixture warmed to 75° as the persulfate was slowly added, and after the addition the mixture was cooled to 10° and ice was added to it. The pH was then adjusted to 9—10 with ammonium hydroxide, and the mixture was extracted three times with 400 ml portions of diethyl ether. The organic layers were combined and washed twice with 400 ml portions of 0.5N sodium hydroxide. The organic layer was then washed with brine, dried over magnesium sulfate, and evaporated under vacuum to obtain 52 g of impure product. It was chromatographed by high performance liquid chromatography, eluting with 5:1 hexane:ethyl acetate, and the desired product was obtained as an oil, amounting to 25 g.

Example 6

3,6-dichloro-4-(1-chloromethylethyl)pyridazine

Five g of the product of Preparation 5 was combined with 3.5 g of sulfuryl chloride and 25 mg of benzoyl peroxide in 25 ml of carbon tetrachloride at ambient temperature under nitrogen, and a 250-watt infrared reflector light was placed close to the flask. The mixture was stirred with irradiation for 3.5 hours, by which time it had warmed to 62° from the lamp. The mixture was then evaporated under vacuum to an oil, which was purified by chromatography on a high performance preparative instrument, eluting with 9:1 hexane:ethyl acetate. Evaporation of the product-containing fractions gave 0.70 g of the desired product. Its elemental analysis was as follows.

Theory: C, 37.28; H, 3.13; N, 12.42;
Found: C, 37.07; H, 3.04; N, 12.15.

Preparation 6

3,6-dichloro-4-*t*-butylpyridazine

A 375 g portion of 3,6-dichloropyridazine was slurried with 578 g of pivalic acid in 367 g of sulfuric acid and 1500 ml of water, and the mixture was warmed to 40°. Then 48.2 g of silver nitrate was added, and the mixture was heated to 62°. To it was added 1 kg of ammonium persulfate in 2 liters of water over a period of 1 hour. The temperature rose exothermically, and was controlled at 80° maximum. After the addition, the mixture was stirred for 15 minutes, and was then cooled to 15° with an ice-water bath. The mixture was then cooled further by the addition of ice, and its pH was adjusted to 9 with ammonium hydroxide. It was then stirred vigorously for 1 hour, while gummy material was scraped from the sides of the vessel as needed. It was then filtered, and the solids were washed with 2 liters of water and dried on the filter pad. The solids were then slurried in 5 liters of diethyl ether and the slurry was filtered. The filtrate was washed three times with 500 ml portions of 1N sodium hydroxide, and the washes were combined and extracted with 500 ml of diethyl ether. That ether was combined with the first ether filtrate, and was washed with 500 ml of brine. The organic layer was dried over magnesium sulfate and carbon treated at the reflux temperature. It was then cooled and filtered through diatomaceous earth, and the filtrate was evaporated under vacuum to obtain 449 g of the desired intermediate.

Example 7

3,6-dichloro-4-[1,1-bis(chloromethyl)-2-chloroethyl]pyridazine

A 5.5 g portion of the product of Example 1 was combined with 9.3 g of sulfuryl chloride and 10 mg of benzoyl peroxide in 15 ml of carbon tetrachloride, and the mixture was irradiated with an infrared lamp as described above in Example 6. Stirring and irradiation was continued for 96 hours at about 40°, and additional sulfuryl chloride was added from time to time, until a total of 37.2 g was added. After the 96 hours, the mixture was evaporated under vacuum to obtain 6.3 g of oil, which was purified by high performance liquid chromatography. The product-containing fractions were combined with similar fractions from another batch, and that mixture was purified again by chromatography, eluting with 10:1 heptane:ethyl acetate, to obtain 0.23 g of the desired product, m.p. 111—112°. The elemental analysis was as follows.

Theory: C, 31.15; H, 2.29; N, 9.08; Cl, 57.47;
Found: C, 31.00; H, 2.22; N, 9.35; Cl, 57.67.

Example 8

3,6-dichloro-4-(1-chloromethyl-2-chloro-1-methylethyl)pyridazine

Two g of the product of Preparation 6 above was combined with 2.6 g of sulfuryl chloride and 50 mg of benzoyl peroxide in 2.5 ml of carbon tetrachloride, and the mixture was stirred with irradiation from a 250-watt infrared reflector lamp for 2 hours. Then 0.66 g of additional sulfuryl chloride was added, and the reaction was continued for another 2 hours. The mixture was then evaporated under vacuum to obtain 2.3 g of oil, which was chromatographed by high performance liquid chromatography, eluting with 9:1 heptane:ethyl acetate. About 0.9 g of the desired product in impure form was collected, and was re-chromatographed with 19:1 heptane:ethyl acetate. About 0.6 g of impure product was obtained, which was retreated with 0.67 g of sulfuryl chloride and benzoyl peroxide, with irradiation as before. The mixture was treated under the lamp at 60° for 20 minutes, and was then set in the freezer overnight. It was then evaporated to a colorless oil under vacuum, and the oil was chromatographed by high performance liquid

chromatography, eluting with dichloromethane, to obtain 100 mg. of the desired product, m.p. 92—94°. The elemental analysis was as follows.

Theory: C, 35.07; H, 2.94; N, 10.22;
Found: C, 35.34; H, 3.00; N, 10.48.

Example 9

3,6-dichloro-4-(1-chloromethyl-2,2-dichloro-1-methylethyl)pyridazine

Fifty g of the product of Preparation 6 above was combined with 99 g of sulfuryl chloride and 25 mg of benzoyl peroxide in 125 ml of carbon tetrachloride, and the mixture was irradiated with an infrared lamp as described in the examples above. After 20 hours, an additional 99 g of sulfuryl chloride was added, and irradiation was continued for 7 hours more. The temperature of the reaction mixture was in the range 58—61° during the process. It was then cooled and evaporated to dryness under vacuum, and the resulting oil was purified by high performance liquid chromatography, eluting with 10:1 heptane:ethyl acetate, to obtain 1.76 g of the desired product, m.p. 82—85°. Its elemental analysis was as follows.

Theory: C, 31.15; H, 2.29; N, 9.08; Cl, 57.47;
Found: C, 31.23; H, 2.26; N, 9.28; Cl, 57.73.

Example 10

3,6-dichloro-4-(1-iodomethyl-1-methylethyl)pyridazine

A 10.3 g portion of the product of Preparation 6 was combined with 11.25 g of N-iodosuccinimide and 100 mg of benzoyl peroxide in 500 ml of carbon tetrachloride, and the mixture was irradiated with a 250-watt infrared lamp about 15 cm from the Pyrex flask. The mixture was stirred under gentle reflux for about 1.25 hours, and then 10 drops of sulfuryl chloride were added. After 2.25 hours, 20 drops more of sulfuryl chloride were added, and reflux was continued. After 4.5 hours, a second lamp of the same type was also placed 15 cm from the flask. After 7.25 hours, another 5 g of N-iodosuccinimide was added and reaction was continued for a total of 25 hours, when the mixture was cooled and filtered. The filtrate was concentrated under vacuum to 20.9 g of oily residue, which was chromatographed on 200 g of silica gel, eluting with hexane:diethyl ether. The initial ratio of the solvents was 50:1, changing to 6:1 and finally to 1:1. The product-containing fractions were combined and concentrated, and the residue was crystallized from diethyl ether/heptane to obtain 0.65 g of product, m.p. 104—106°. Re-chromatography of the mother liquor gave about 1 g of crystalline product, which was combined with the first crop and recrystallized to obtain 1.1 g of pale yellow needles, m.p. 106—108°. The elemental analysis was as follows.

Theory: C, 29.03; H, 2.74; N, 8.46;
Found: C, 29.26; H, 2.80; N, 8.25.

Example 11

3,6-dichloro-4-(1-chloromethyl-1-methylethyl)-5-methylpyridazine

Ten g of the product of Example 1 was slurried with 120 ml of water, 10 g of acetic acid, 6.1 g of sulfuric acid and 3.6 g of silver nitrate, and the mixture was heated to 60°. To it was added a solution of 28.6 g of ammonium persulfate in 50 ml of water. The reaction was exothermic, and the temperature reached 84° upon addition of the first small amount of persulfate. The mixture was then cooled to 60° and the rest of the persulfate was added over 20 minutes, holding the temperature at 60—65°. After the addition, the mixture was stirred for 1 hour while it cooled to ambient temperature, and it was then chilled to 10° and its pH was adjusted to 7—8 with ammonium hydroxide. It was then allowed to stand, and the clear aqueous supernatant was poured off. The remaining material was slurried twice with 200 ml portions of diethyl ether. The ether was combined with the aqueous layer, and shaken, and the layers were separated. The aqueous layer was re-extracted with 200 ml of additional diethyl ether, and all of the organic layers were combined and washed with 100 ml of brine and dried over magnesium sulfate. The solvent was then removed under vacuum, leaving an oil which appeared, under nuclear magnetic resonance analysis, to be mainly starting material.

The oil was combined with 10 g of acetic acid, 3.6 g of silver nitrate and 6.1 g of sulfuric acid in 70 ml of water, and 28.6 g of ammonium persulfate in 60 ml of water was slowly added, as above. When the addition was complete, the mixture was cooled to 40°, and additional portions of the same amounts of acetic acid, silver nitrate and ammonium persulfate were added. The mixture was stirred for 1 hour more, and was worked up as described in the first step of this example. About 6 g of oil was obtained, which was separated by high performance liquid chromatography, eluting with 5:1 heptane:ethyl acetate. The product-containing fractions were combined, and the residue of them was rechromatographed, eluting this time with 9:1 heptane:ethyl acetate to obtain 0.53 g of the desired product, m.p. 56—58°. Its elemental analysis was as follows.

Theory: C, 42.63; H, 4.37; N, 11.05;
Found: C, 42.93; H, 4.51; N, 10.93.

Example 12

3-chloro-4-(1-chloromethyl-1-methylethyl)-6-(furan-2-ylmethoxy)pyridazine

A 0.66 g portion of 50% sodium hydride in mineral oil was washed with hexane, 40 ml of anhydrous dimethylformamide was added, and the suspension was cooled to 10°. To it was added 1.47 g of furan-2-yl-methanol, and the mixture was warmed and stirred at ambient temperature for 1.5 hours. Then 3.0 g of the product of Example 1 was added over a period of 5 minutes, dissolved in 5 ml of anhydrous dimethylformamide. The mixture was warmed to 45° exothermically, and it was then heated to 60° and stirred at that temperature for 1.5 hours. Then it was cooled and stirred at ambient temperature for 16 hours, and was evaporated under vacuum to a dark oil. The oil was slurried in 300 ml of water, and the aqueous mixture was extracted with 200 ml of ethyl acetate. The organic layer was separated, washed with 50 ml of brine and dried over magnesium sulfate. The solvent was then removed under vacuum to obtain 2.8 g of oil, which was chromatographed by high performance liquid chromatography, eluting with dichloromethane, to obtain 1.15 g of impure product. It was re-chromatographed, eluting with 5:1 hexane:ethyl acetate, to obtain 300 mg of the desired product, the elemental analysis of which was as follows.

Theory: C, 51.85; H, 4.69; N, 9.30;
Found: C, 51.63; H, 4.56; N, 9.31.

Example 13

3,6-dichloro-4-(1-chloromethyl-1-methylethyl)pyridazine, $N^2$-oxide

Example 14

3,6-dichloro-4-(1-chloromethyl-1-methylethyl)pyridazine, $N^1$-oxide

Six g of the compound of Example 1 was slurried in 50 ml of dichloromethane, and 8 g of 3-chloroperoxybenzoic acid was added. The mixture was stirred at the reflux temperature for 8 hours, and was then evaporated under vacuum to a solid. It was taken up in a minimum amount of carbon tetrachloride:ethyl acetate, and poured over 300 ml of silica gel. Elution of the column with 2:1 heptane:ethyl acetate produced about 6 g of an impure mixture of both oxides. The mixture was separated by high performance liquid chromatography, eluting with 5:1 heptane:ethyl acetate. About 0.4 g of the compound of Exampe 13 was obtained, m.p. 90—92°. Its elemental analysis was as follows.

Theory: C, 37.60; H, 3.55; N, 10.96;
Found: C, 37.88; H, 3.65; N, 11.21.

The chromatography also separated about 0.4 g of the product of Example 14, m.p. 106—107°.

Theory: C, 37.60; H, 3.55; N, 10.96;
Found: C, 37.72; H, 3.33; N, 10.70.

Preparation 7

3,6-dibromopyridazine

A 22.8 g portion of 3,6-dioxopyridazine (maleic acid hydrazide) was combined with 115 g of phosphorus oxybromide and 150 ml of benzene and the mixture was stirred for 3 hours at 70—80°. It was cooled and diluted with 100 ml of diethyl ether, and the mixture was poured over ice and made slightly basic with ammonium hydroxide. The organic layer was separated, and was washed with water and dried over sodium sulfate. It was then concentrated under vacuum, and the white residue was recrystallized from cyclohexane to obtain 6.4 g of the desired intermediate, m.p. 116—117°.

Theory: C, 20.28; H, 0.85; N, 11.78;
Found: C, 20.44; H, 0.86; N, 11.81.

Example 15

3,6-dibromo-4-(1-bromomethyl-1-methylethyl)pyridazine

Three g of 3,6-dibromopyridazine, 1 g of silver nitrate, 3.2 g of 2,2-dimethyl-1,3-propanediol, 7 ml of water and 1.9 g of concentrated sulfuric acid were stirred at 30° while 5.8 g of ammonium persulfate dissolved in 15 ml of water was added dropwise. The temperature rose to 60°. The mixture was then cooled and extracted with 100 ml of dichloromethane. The organic layer was washed with 10 ml of water and dried over magnesium sulfate. The solvent was removed under vacuum, and the resulting solid was chromatographed on silica gel, eluting with 3:1 hexane:ethyl acetate in a high performance liquid chromatography device. The product-containing fractions were combined and evaporated under vacuum, and the product was recrystallized from benzene/hexane to obtain 1.4 g of 3,6-dibromo-4-(1-hydroxymethyl-1-methylethyl)-pyridazine, m.p. 140—141°.

A 9.3 g portion of the above intermediate, obtained from successive reactions, was stirred with 20 ml of pyridine and 8.6 g of *p*-toluenesulfonyl chloride at ambient temperature overnight. The mixture was then poured into ice-water, diethyl ether was added, and the resulting precipitate was collected. The solids were dissolved in ethyl acetate, and the solution was washed with 0.5N hydrochloric acid and then with aqueous sodium bicarbonate. The organic layer was dried over magnesium sulfate, and was evaporated under vacuum. The solids were recrystallized from ethyl acetate/hexane to obtain 10 g of 3,6-dibromo-4-(1-methyl-1-*p*-toluenesulfonyloxymethylethyl)pyridazine, m.p. 119—121°.

To 4.6 g of the above intermediate was added 10 ml of dimethylsulfoxide and 1.7 g of lithium bromide. The mixture was stirred at 110° for 2.5 hours, and it was then cooled and poured into ice-water. The resulting white gummy solid was dissolved in diethyl ether, and the solution was washed with water and

dried. The solvent was removed under vacuum to leave an oil, which was purified by high-performance liquid chromatography over silica gel, eluting with 4:1 heptane:ethyl acetate. About 600 mg of product, m.p. 125—126°, was collected.

Theory: C, 25.77; H, 2.43; N, 7.51;
Found: C, 26.02; H, 2.30; N, 7.53.

Example 16

3-bromo-6-chloro-4-(1-chloromethyl-1-methylethyl)pyridazine

Four g of 3,6-dibromo-4-(1-methyl-1-*p*-toluenesulfonyloxymethylethyl)pyridazine was combined with 8 ml of dimethylsulfoxide and 1.5 g of lithium chloride and the mixture was stirred for 2 hours at 90—100°. It was then poured into ice-water, and the white solid was collected and dissolved in diethyl ether. The solution was washed with water, dried, and evaporated under vacuum. The residue was recrystallized from hexane to obtain about 2.1 g of the desired product, m.p. 79—81°. Its nuclear magnetic resonance spectrum, run in $CDCl_3$ on a 60 mHz instrument, showed characteristic features at δ 1.65, s, 6H; 4.10, s, 2H; 7.45, s, 1H.

Preparation 8

3,6-dimethylpyridazine

Fifty g of 2,5-dimethylfuran was combined with 156 g of anhydrous sodium carbonate and 1,050 ml of methanol and the mixture was chilled to −15°. To it was added, over 45 minutes, 83.1 g of bromine while the temperature was held constant. The mixture was then stirred while it warmed to ambient temperature, and the liquid portion was decanted into a 4-liter separatory funnel containing 2 liters of brine. The liquid was extracted twice with 800 ml portions of dichloromethane, and the organic layers were combined and washed with 100 ml of fresh brine. Then the organic portion was dried over magnesium sulfate, filtered and evaporated under vacuum to obtain an oily residue which was vacuum distilled at 45—48°, at 8—11 mm pressure, to obtain 49 g of 2,5-dimethoxy-2,5-dimethylfuran.

A 54 g portion of the above intermediate, obtained by successive reactions, was combined with 68 ml of 1% aqueous acetic acid, and then 18.5 ml of 85% hydrazine hydrate was added over a period of 25 minutes. The temperature of the mixture increased to 65° while the addition was made, and the mixture was then heated to 78° and stirred overnight at that temperature. It was then cooled and filtered. The filtrate was evaporated under vacuum to obtain a brown oil, which was distilled. The product remained in the distillation vessel, and was purified by high-performance liquid chromatography, eluting with ethyl acetate, to obtain 16.3 g of 3,6-dimethylpyridazine.

Example 17

4-(1-chloromethyl-1-methylethyl)-3,6-dimethylpyridazine

A 23.9 g portion of 2,2-dimethyl-1,3-propanediol, 88 ml of water, 11.3 g of 3,6-dimethylpyridazine, 3.6 g of silver nitrate and 12.3 g of concentrated sulfuric acid were combined at ambient temperature, and to the mixture was added 41.8 g of ammonium persulfate dissolved in 68 ml of water. The addition was dropwise over a period of only 15 minutes. The reaction temperature rose to 75°, and the mixture was stirred at that temperature for 30 minutes. The reaction mixture was then worked up substantially as described in the examples above to obtain 1.2 g of 4-(1-hydroxymethyl-1-methylethyl)-3,6-dimethylpyridazine.

A 2.2 g portion of the above intermediate, obtained from successive reactions, was reacted with 3.5 g of *p*-toluenesulfonyl chloride in 40 ml of dry pyridine, and the mixture was worked up substantially as described in Example 15 above to obtain 2.6 g of 3,6-dimethyl-1-(1-methyl-1-*p*-toluenesulfonyloxymethy-ethyl)pyridazine, m.p. 121—122°.

One g of the above intermediate was dissolved in 10 ml of dimethylsulfoxide, and 0.5 g of lithium chloride was added. The mixture was heated to 110° and held at that temperature for 2 hours. It was then cooled to about 40°, and poured into 300 ml of water. The mixture was salted out with sodium chloride, and was extracted twice with 150 ml portions of diethyl ether. The organics were evaporated under vacuum, and the resulting oil was slurried in 10 ml of water and extracted with 200 ml of warm heptane. The organic layer was then filtered through phase separation paper, and was evaporated under vacuum to obtain 0.35 g of an oil, which crystallized on standing. It was then dissolved in ethyl acetate and poured over silica gel and evaporated again to obtain 0.21 g of the desired product, m.p. 57°—59°.

Theory: C, 60.45; H, 7.61; N, 14.10;
Found: C, 60.74; H, 7.40; N, 14.02.

Example 18

4-(1-bromomethyl-1-methylethyl)-3,6-dimethylpyridazine

A 1.0 g portion of 3,6-dimethyl-1-(1-methyl-1-*p*-toluenesulfonyloxymethylethyl)pyridazine was dissolved in 10 ml of dimethylsulfoxide under nitrogen, and 0.52 g of lithium bromide was added. The mixture was held at 110° for 2.5 hours, and was poured into 300 ml of water, which was then saturated with sodium chloride. The solution was extracted twice with 150 ml portions of diethyl ether, and the combined organic layers were washed with brine and dried over magnesium sulfate. The organic solution was then

evaporated under vacuum to obtain a residue of impure product, which was recrystallized from diethyl ether/heptane to obtain 0.43 g of the desired product, m.p. 45°—46°.

Theory: C, 49.40; H, 6.22; N, 11.52;
Found: C, 49.66; H, 5.96; N, 11.61.

Example 19

3,6-dichloro-4-(chloromethyl)dimethysilylpyridazine

Twenty g of 3,6-dichloropyridazine was combined with 50 ml of nitrogen-bubbled acetonitrile, 29.2 g of (chloromethyl)dimethylsilane and 39.2 g of di-*t*-butylperoxide. The mixture was kept under nitrogen, and was exposed overnight to a 275 watt infrared lamp. The final temperature was 44°. The lamp was then moved closer, and the temperature increased to 54° while irradiation continued for 5 hours more. The mixture was then evaporated under vacuum, and the residue was dissolved in 100 ml of boiling carbon tetrachloride and the solution was filtered. The filtrate was then subjected to high-performance liquid chromatography, eluting with 7:1 heptane:ethyl acetate, and the product-containing fractions were combined to obtain 231 mg of the desired product as an oil. Analysis by mass spectroscopy showed the desired molecular ion of weight 254, and the expected ion of weight 205, resulting from loss of the chloromethyl group in the analysis.

Preparation 9

3-chloro-6-cyanopyridazine

Ninety g of 6-oxo-3-pyridazinecarboxylic acid was slurried with 270 ml of phosphorus oxychloride and 1 ml of dimethylformamide under nitrogen, and the mixture was stirred under reflux for 1.5 hours. It was then evaporated under vacuum, and the residue was poured into 3 liters of 28% aqueous ammonia. The aqueous mixture was then poured into a 22 liter flask, and was extracted three times with 4-liter portions of ethyl acetate. After the first extraction, the pH was adjusted to 7 with hydrochloric acid. The extracts were combined and evaporated under vacuum to obtain 27.7 g of 3-chloro-6-pyridazinecarboxamide.

The above intermediate was slurried with 300 ml of phosphorus oxychloride under nitrogen, and the mixture was held at 80°—90° for 2.5 hours. It was then evaporated under vacuum, and the residue was slowly added to 800 ml of warm water. The aqueous mixture was extracted twice with 800 ml portions of ethyl acetate, and then was extracted three times with 800 ml portions of diethyl ether. All of the organic layers were combined, and washed with brine. The washed organic layer was dried over magnesium sulfate and evaporated under vacuum. The residue was taken up in diethyl ether and poured over 600 ml of silica gel, eluting with diethyl ether. The product-containing fractions were collected and evaporated under vacuum to obtain 13 g of the desired intermediate.

Example 20

3-chloro-4-(1-chloromethyl-1-methylethyl)-6-cyanopyridazine

Example 21

3-chloro-4-[1,1-bis(chloromethyl)ethyl]-6-cyanopyridazine

Five g of 3-chloro-6-cyanopyridazine was combined with 8 g of pivalic acid, 1.2 g of silver nitrate, 5.26 g of concentrated sulfuric acid and 20 ml of water, and the mixture was heated to 60°. A 14.3 g portion of ammonium persulfate was dissolved in 28 ml of water, and the solution was added to the first mixture over 15 minutes, while the temperature was held between 60°—75°. Then the mixture was heated to 80° for 20 minutes, and was chilled to 15° and its pH was adjusted to 9 with ammonium hydroxide. The water layer was then removed, and the remaining solids were washed three times with 200 ml portions of diethyl ether. The ether was added to the water layer, and the organic extract was separated. The water layer was extracted again with diethyl ether, and all of the organics were combined and washed twice with 100 ml portions of 1N sodium hydroxide. The organic layer was then washed with brine and dried over magnesium sulfate. It was then evaporated under vacuum and purified by high-performance liquid chromatography, eluting with 6:1 heptane:ethyl acetate to obtain 2.7 g of 3-chloro-4-*t*-butyl-6-cyanopyridazine, m.p. 118—119°.

The above intermediate was added to 10 ml of carbon tetrachloride and 1.9 g of sulfuryl chloride under nitrogen at ambient temperature. The mixture was irradiated with a 275 watt sun lamp for about 15 minutes. The temperature of the mixture reached 55°. The mixture was then evaporated under vacuum, and the residue was purified by high-performance liquid chromatography, eluting with 10:1 heptane:ethyl acetate. The product-containing fractions were combined and evaporated under vacuum to obtain 0.87 g of the product of Example 20, m.p. 75°—76°.

Theory: C, 46.98; H, 3.94; N, 18.26;
Found: C, 47.07; H, 3.80; N, 18.00.

The fractions which came off before those containing the product above were combined and evaporated to obtain 100 mg of the product of Example 21, m.p. 66—68°.

Example 22

3,6-dichloro-4-(2-chlorocyclohexyl)pyridazine

A 17.9 g portion of 3,6-dichloropyridazine was combined with 120 ml of cyclohexene, 4.1 g of silver nitrate, 11 ml of concentrated sulfuric acid and 60 ml of acetonitrile in 120 ml of water at 50°. To that mixture was then added 41.1 g of ammonium persulfate dissolved in 60 ml of water, in 5 minutes. The mixture was stirred for 2 hours more, and was then diluted with 300 ml of dichloromethane and filtered. The filtrate was extracted three times with 300 ml portions of dichloromethane, and the combined organics were washed with 50 ml of saturated aqueous sodium bicarbonate and 50 ml of water. The organic layer was then filtered through phase separation paper and concentrated under vacuum to obtain 31.7 g of tan solid. The residue was crystallized from ethyl acetate to obtain 11.5 g of 3,6-dichloro-4-(2-hydroxycyclohexyl)pyridazine, m.p. 141—143°. A second crop of 6.6 g of product, m.p. 139—140°, was also collected.

Five g of the above first-crop intermediate was added to 5 ml of pyridine, 50 ml of toluene and 4.5 ml of thionyl chloride, and the mixture was stirred for 2 hours at 80°. It was then cooled and diluted with 100 ml of water, and was extracted three times with 150 ml portions of diethyl ether. The combined organics were washed with 25 ml portions of saturated aqueous sodium bicarbonate and water. The organic layer was then dried over magnesium sulfate and evaporated under vacuum. The resulting oil was filtered through 15 g of silica gel with 100 ml of diethyl ether and was crystallized. A total of 1.8 g of product, m.p. 93—95°, was collected in two crystallizations.

Theory: C, 45.23; H, 4.18; N, 10.55;
Found: C, 44.99; H, 3.93; N, 10.62.

Preparation 10

3-chloro-6-methylpyridazine

A 245 g portion of levulinic acid was added to 3 liters of ethanol and 69 g of anhydrous hydrazine was added. The mixture was stirred under reflux for 3 hours, and the solvent was removed under vacuum. The residue was slurried with 400 ml of ethyl acetate and the solids were separated by filtration to obtain 259 g of 3-methyl-4,5-dihydro-6-oxopyridazine.

The above intermediate was added to 2.5 liters of acetic acid and the mixture was heated with stirring to 100° and was held at 100—114° while 338 g of bromine was added over a period of 30 minutes. It was stirred under reflux for 1 hour after the addition, and then it was cooled and evaporated under vacuum, and 1.5 liters of water was added. The mixture was filtered, and the filtrate was chilled and was filtered again to obtain a total of 202 g of 3-methyl-6-oxopyridazine.

The above intermediate was slowly added to 1,280 ml of phosphorus oxychloride, and the mixture was slowly heated to the reflux temperature and held at that temperature for 1 hour. It was then evaporated under vacuum, and the oily residue was added to water with stirring. The pH was adjusted to 6, and the aqueous mixture was extracted with dichloromethane. The extract was washed with brine, was dried and was evaporated under vacuum. The aqueous layer was re-adjusted to pH 6 and was extracted two times with 1,500 ml portions of dichloromethane. The extracts were combined, washed with brine, dried and evaporated under vacuum to obtain a total of 1.5 g of 3-chloro-6-methylpyridazine.

Example 23

3-chloro-4-(1-chloromethyl-1-methylethyl)-6-methylpyridazine

Ten g of 3-chloro-6-methylpyridazine was combined with 17.8 g of 2,2-dimethyl-1-3-propanediol, 6.6 g of silver nitrate, 9.2 g of concentrated sulfuric acid and 120 ml of water, and the mixture was warmed to 29°. To it was added, dropwise, 31.1 g of ammonium persulfate dissolved in 90 ml of water. The temperature was 65° at the end of the 15-minute addition. The mixture was stirred at about that temperature for 20 minutes more, and was then cooled to ambient temperature and was extracted with dichloromethane. The extract was washed with water and evaporated under vacuum. The residue was recrystallized from 80 ml of toluene and then from 100 ml of dichloromethane. A total of 3.9 g of 3-chloro-4-(1-hydroxymethyl-1-methylethyl)-6-methylpyridazine was obtained, m.p. 151—153°.

A 1.5 g portion of the above intermediate was reacted with 2.2 g of *p*-toluenesulfonyl chloride under nitrogen at ambient temperature in pyridine, as described in examples above, to obtain 1.6 g of 3-chloro-4-(1-methyl-1-*p*-toluenesulfonyloxymethylethyl)-6-methylpyridazine.

One g of the above intermediate was dissolved in 10 ml of dimethylsulfoxide and was reacted with 0.24 g of lithium chloride at 110°, and was worked up as described in the examples above to obtain 0.42 g of the desired product, m.p. 41—42°.

Theory: C, 49.33; H, 5.62; N, 12.78;
Found: C, 49.55; H, 5.79; N, 12.56.

Example 24

4-(1-bromomethyl-1-methylethyl)-3-chloro-6-methylpyridazine

A 0.6 g portion of 3-chloro-4-(1-methyl-1-*p*-toluenesulfonyloxymethylethyl)-6-methylpyridazine was dissolved in 10 ml of dimethylsulfoxide and was reacted with 0.3 g of lithium bromide at 110°, as described in examples above, to obtain 200 mg of the desired product, a light yellow oil, the identity of which was

confirmed by mass spectroscopy. A molecular ion of weight 262 was observed, as was the ion of weight 169 which resulted from loss of the bromomethyl group from the compound.

Example 25

3,6-dichloro-4-(2,2-dichloro-1-methylcyclopropyl)pyridazine

A 3.5 g portion of 3,6-dichloropyridazine was slurried with 4.0 g of 2,2-dichloro-1-methylcyclopropanecarboxylic acid, 15 ml of water, 3.4 g of concentrated sulfuric acid in 20 ml of water, and 4.0 g of silver nitrate, and the mixture was heated to 65° with rapid stirring. To it was added 11.9 g of ammonium persulfate in 20 ml of water, over 20 minutes, while the temperature was held at 70°—76°. The mixture was stirred 15 minutes after the addition and cooled to 10°. Its pH was adjusted to 8.0 with ammonium hydroxide, and the water layer was then decanted off. The remaining solids were washed twice with 100 ml portions of diethyl ether, and that ether was then used to extract the water layer. The organic layer was washed with 1N sodium hydroxide and with brine, and dried. The solvent was removed under vacuum, and the residue was purified by chromatography, eluting with 3:1 heptane:ethyl acetate. The product-containing fractions were combined and evaporated, and the residue was recrystallized from heptane, to obtain 0.52 g of the desired product, m.p. 78—79°.

Theory: C, 35.33; H, 2.22; N, 10.30;
Found: C, 35.16; H, 2.21; N, 10.24.

The compounds of the present invention are particularly valuable when used in the method of this invention, which is a method of reducing the adverse effects of diseases of plants caused by fungi of the Phycomycete group. The Phycomycetes are a well-known class of fungi which cause many disastrous diseases, including late blight of potato. The following typical plant diseases are caused by Phycomycetes and are mentioned, with their causative organisms, to assure that the reader understands the uses of the compounds.

black wart of potato — *Synchtrium endobioticum*
brown spot of maize — *Physoderma zeaemaydis*
crown wart of alfalfa — *Physoderma alfalfae*
root rot of pea — *Aphanomyces euteiches*
Pythium damping-off,
root rot, stem rot,
soft rot and stalk rot
of many crops — *Pythium arnhenomanes, P. aphanidermatum, P. ultimum, P. debaryanum, P. splendens, P. scleroteichum*
milo disease of sorghum — *Periconia circinata*
late blight of potato and tomato — *Phytophthora infestans*
fruit rot of pear and apple — *Phytophthora cactorum*
brown rot of lemon — *Phytophthora citrophthora*
tomato root rot — *Phytophthora cryptogea*
rot of pepper — *Phytophthora capsici*
coconut bud rot — *Phytophthora palmivova*
black shank of tobacco — *Phytophthora parasitica var. nicotiniae*
blue mold of tobacco — *Peronospora tabacina*
root rot of cauliflower — *Phytophthora megasperma*
root rot of avocado — *Phytophthora cinnamoni*
white rust — *Albugo candida, A. occidentalis, A. ipomoeaepanduraneae, A. minor, A. tragopogonis*
downy mildew of grass — *Sclerospora graminicola*
downy mildew of grape — *Plasmopara viticola*
downy mildew of onion — *Peronospora destructor*
downy mildew of cucurbits — *Pseudoperonospora cubensis*
downy mildew of lettuce — *Bremia lactucae*

The method of the present invention is carried out by applying a compound of the invention to a plant to be protected from such fungi, or to the soil in which the plant grows. The compounds are effective when applied either before or after infection by such a fungus. The tests reported above show that the interval between treatment and infection can be as much as 7 to 9 days, or even longer, depending on the circumstances.

The method of the invention is effective against Phycomycetes which infect both the foliage and the roots of plants. Accordingly, depending on the disease from which the plants are expected to suffer, the compounds can usefully be applied to the foliage or to the soil in which the plants grow. Further, the

compounds can beneficially be applied to seed, in the form of a seed treatment or coating, before the seed is planted. Alternatively, a composition containing a compound of the invention can be applied to the soil in a small area around the seed, by an applicator which is part of the planter, to assure that the compound is in close contact with the soil in which the plant is actually rooted.

Alternatively, when a compound is to be applied to the soil, it may be advantageous to carry the compound, or the composition containing the compound, into the soil with a large amount of water to assure contact with the roots and the soil immediately around the roots.

When a plant protectant is applied to foliage, it is customary to measure the dosage of the compound by its concentration in the dispersion which is actually applied. The reason is that the amount of the dispersion retained on the leaves is essentially constant, and depends primarily on the area of the foliage. Thus, the amount of compound applied can be varied only by varying the concentration of it in the dispersion. In general, the concentration of compounds of the present invention in spray dispersions is in the range of from about 1 part per million (ppm) to about 1,000 ppm, depending on the identity of the compound, the severity of the infection which is present or is expected, the organism of primary concern, the weather and other factors known to plant pathologists. More preferable concentrations are in the range from about 5 ppm to about 1,000 ppm, still more preferably in the range from about 10 ppm to about 500 ppm.

When a compound is to be applied to the soil, it is often convenient to express the dosage in terms of amount of compound per unit of area. The present compounds are usefully applied to the soil at rates from about 0.1 to about 10 pounds per acre, more preferably at rates from about 0.5 to about 5 pounds per acre. The same factors mentioned above are material to choosing a soil application rate, and in addition the soil type, moisture content and organic content are pertinent to the decision as well.

It will be understood that higher application rates are needed in field-grown crops than are needed in the greenhouse. The compounds are useful in both environments, and the reader who is knowledgeable in plant protection will choose application rates in the higher ranges for field use, and in the lower ranges for greenhouse use. The many tests reported above are helpful in determining application rates.

In earlier days, it was common to apply fungicides and other plant protectants as dusts. Such applications are now seldom used because they are inefficient. However, dusts of the present compounds can be used, and should contain the active ingredient in concentrations in the range of from about 0.5% to about 5%.

It is usual to apply plant protectants to a crop several times in the course of a season. The same practice should be followed in using the present compounds. The interval between applications depends on the weather, the severity of infection and the rate at which the crop is growing. In general, intervals of from several days to a few weeks are appropriate. Preferably, the interval should be in the range of about 5—15 days.

The above preferred concentrations and dosage amounts of the compounds are given for the guidance of the practitioner, but it will be understood by one who has read the tests above that application of a Phycomycete-inhibiting amount of a compound reduces the adverse effects of the disease, even though only a part of the Phycomycete population may be killed by the compound. The term "Phycomycete-inhibiting amount" is used here to describe an amount which is sufficient to reduce the adverse effects of a Phycomycetous fungus. The term "reducing the adverse effects" refers to weakening the pathogen sufficiently that its reproduction rate and its vigor are decreased, with the result that the express signs of the disease, and the resulting injury to the host plant, are decreased.

Agricultural chemists and farmers know of a great many adjuvants used to improve the spreading and sticking of agricultural compounds on foliage. Such additives are customarily sold under trade-names, such as Agrimul 26B, Agriwet FR, Codicide Oil, Joncryl 77, Enhance, Nufilm 17, Tack System 5, Herb-Ad, Ad-Wet, Pen-A-Trate, Bivert and many others. Such conventional adjuvants, which are commercially available to farmers, may be used in conjunction with foliar applications of the present compounds and will improve their activity and extend the period between applications of the compound.

It has been discovered, however, that a particular group of adjuvants have a remarkable effect on the activity of the present compounds. These preferred adjuvants are quaternary ammonium salts of trialkoxysilyl alkylamines. The preferred adjuvant is sold by Petrarch Systems, Inc., Bristol, Pennsylvania under the code name C—09745. It is octadecyldimethyl-[3-(trimethoxysilyl)propyl]ammonium chloride.

Use of an adjuvant such as C—09745 both reduces the necessary application rate of a compound of this invention and extends its residual life, so that the interval between applications may be extended. It is advisable to add from about 0.01% to about 0.1% of an adjuvant such as C—09745 to the complete spray mixture. The higher part of the range of rates is preferred, such as from about 0.05% to about 0.1%.

It will be seen that considerably more adjuvant than compound is usually used. Thus, it is not preferred to include the adjuvant in a composition according to the invention, but, rather, simply to add the adjuvant to the spray tank immediately before application. Adjuvants such as C—09745 are not particularly stable in water, so it is not advisable to prepare a spray mixture containing such an adjuvant in advance of its use.

## The Combinations

An important embodiment of the present invention is a group of fungicidal combination compositions and fungicidal methods which comprise a compound of the invention in combination with a

dithiocarbamate fungicide. The general formula of the dithiocarbamates was described above in the Summary of the Invention.

Certain classes of the dithiocarbamates are particularly preferred for use in the present combinations. It will be understood that the following preferred classes of dithiocarbamates may be combined with any of the preferred classes of compounds of the invention to obtain preferred classes of combinations.

A preferred group of dithiocarbamates is that wherein M is a zinc ion, a manganese ion or a coordination complex of both. Another preferred class is that wherein $M^1$ is a sodium or a ferric ion. Further preferred classes of dithiocarbamates are those wherein $R^8$ is ethylene; wherein $R^9$ is hydrogen or methyl; and that wherein $R^{10}$ is methyl.

Particular dithiocarbamate fungicides which are preferred are ferbam, nabam, maneb, mancozeb, zineb, and ziram. Maneb and mancozeb are particularly preferred.

The combinations are used on the same plants, and for the control of the same diseases, as are the compounds of the invention. Use of the combinations, particularly combinations including the preferred dithiocarbamates with the preferred compounds of the invention, extends the interval between applications of fungicide, and allows the use of lower application rates. Thus, use of a compound of the invention in combination with a dithiocarbamate allows acceptable Phycomycete control for a period of about 7—14 days, depending on the weather and the severity of the infection which is encountered.

When a combination is used in the field, the preferred foliar application rate is in the range of from about 50 to about 250 g/ha of the compound of the invention, plus from about 1,200 to about 1,500 ppm of the dithiocarbamate. If the application is measured by concentration in the spray mixture, rather than in weight per land area, the absolute numbers are the same — about 50—250 ppm of compound of this invention plus about 1,200—1,500 ppm of dithiocarbamate. Higher rates can, of course, be used, such as from about 250 to about 1,000 g/ha or ppm of compound of the invention, and about 1,500—2,500 g/ha or ppm of dithiocarbamate. In general, however, it will be found that application rates in the preferred range of rates are effective, and are, of course, more economical and free of side effects than are higher application rates. It has already been shown that the use of adjuvants, particularly the quaternary ammonium silyl adjuvants, increase the activity of the compounds of the invention and extend the period between spray applications. The same fact is true of the combinations with dithiocarbamates, and the use of such adjuvants with the combinations is a particularly preferred embodiment of the invention.

The dithiocarbamate fungicides are very well known in agricultural chemistry, and formulations of them are available as articles of commerce. Such formulations may be used in preparing the present combinations, as by simply adding formulations of the dithiocarbamate and the compound of the invention to a conventional spray tank. Equally, combination formulations may be prepared, in which both compounds are present at a convenient ratio, such as, for example, 10 parts of dithiocarbamate and 1 part of compound of the invention. Such combination formulations offer no particular difficulties to an agricultural chemist and are prepared on the same principles as are formulations of compounds of the invention, which are extensively discussed above.

## Compositions

The compounds of this invention are applied in the form of compositions which are important embodiments of the invention, and which comprise a compound of this invention and a phytologically-acceptable inert carrier. The compositions are either concentrated formulations which are dispersed in water for application, or are dust or granular formulations which are applied without further treatment. The compositions are prepared according to procedures and formulae which are conventional in the agricultural chemical art, but which are novel and important because of the presence therein of the compounds of this invention. Some description of the formulation of the compositions will be given, however, to assure that agricultural chemists can readily prepare any desired composition.

The dispersions in which the compounds are applied are most often aqueous suspensions or emulsions prepared from concentrated formulations of the compounds. Such water-soluble, water-suspendable or emulsifiable formulations are either solids usually known as wettable powders, or liquids usually known as emulsifiable concentrates or aqueous suspensions. Wettable powders, which may be compacted to form wettable granules, comprise an intimate mixture of the active compound, an inert carrier and surfactants. The concentration of the active compound is usually from about 10% to about 90% by weight. The inert carrier is usually chosen from among the attapulgite clays, the montmorillonite clays, the diatomaceous earths, or the purified silicates. Effective surfactants, comprising from about 0.5% to about 10% of the wettable powder, are found among the sulfonated lignins, the condensed naphthalenesulfonates, the naphthalenesulfonates, the alkylbenzenesulfonates, the alkyl sulfates, and nonionic surfactants such as ethylene oxide adducts of alkyl phenols.

Emulsifiable concentrates of the compounds comprise a convenient concentration of a compound, such as from about 10% to about 50% by weight of liquid, dissolved in an inert carrier which is a mixture of water-immiscible organic solvent and emulsifiers. Useful organic solvents include the aromatics, especially the xylenes, and the petroleum fractions, especially the high-boiling napthalenic and olefinic portions of petroleum such as heavy aromatic naphtha. Other organic solvents may also be used, such as the terpenic solvents including rosin derivatives, and complex alcohols such as 2-ethoxyethanol. Suitable emulsifiers for emulsifiable concentrates are chosen from the same types of surfactants discussed above.

Aqueous suspensions comprise suspensions of water-insoluble compounds of this invention, dispersed in an aqueous vehicle at a concentration in the range from about 5% to about 50% by weight. Suspensions are prepared by finely grinding the compound, and vigorously mixing it into a vehicle comprised of water and surfactants chosen from the same types discussed above. Inert ingredients, such as inorganic salts and synthetic or natural gums, may also be added, to increase the density and viscosity of the aqueous vehicle. It is often most effective to grind and mix the compound at the same time by preparing the aqueous mixture, and homogenizing it in an implement such as a sand mill, ball mill, or piston-type homogenizer.

The compounds may also be applied as granular compositions, which are particularly useful for applications to the soil. Granular compositions usually contain from about 0.5% to about 10% by weight of the compound, dispersed in an inert carrier which consists entirely or in large part of clay or a similar inexpensive substance. Such compositions are usually prepared by dissolving the compound in a suitable solvent, and applying it to a granular carrier which has been pre-formed to the appropriate particle size, in the range of from about 0.5 to about 3 mm. Such compositions may also be formulated by making a dough or paste of the carrier and compound, and crushing and drying to obtain the desired granular particle size.

Dusts containing the compounds are prepared simply by intimately mixing the compound in powdered form with a suitable dusty agricultural carrier, such as kaolin clay, ground volcanic rock and the like. Dusts can suitably contain from about 1% to about 10% of the compound.

The following typical formulations of compounds of the invention have been prepared, and are typical of compositions useful in the practice of the present invention.

A. *50% Wettable Powder*

| | |
|---|---|
| Compound of Example 2 | 52.08% |
| Sodium Lauryl Sulfate | 5.00 |
| Purified Silica | 5.00 |
| Lignin Sulfonate | 5.00 |
| Kaolin | 32.92 |

The ingredients were mixed and ground through an air-impact mill.

B. *$1.2 \times 10^2$ kg/$m^3$ (1 lb/gal) Suspension*

| | |
|---|---|
| Compound of Example 1 | 12.5% |
| Precipitated Silica | 1.0 |
| 2% Xanthan Solution | 10.0 |
| Antifoam | 0.2 |
| Lignin Sulfonate | 0.5 |
| Polyfon MT—603 (hydrophobic rosin) | 4.5 |
| Ethylene glycol | 4.5 |
| Tergitol TMN—6 (nonionic surfactant) | 1.0 |
| Water | 65.8 |

The product was ground in an attrition mill.

C. *1.2 × $10^2$ kg/$m^3$ (1 lb/gal) Suspension*

| | |
|---|---|
| Compound of Example 1 | 12.5% |
| Precipitated Silica | 1.0 |
| Antifoam | 0.2 |
| 2% Xanthan Solution | 10.0 |
| Makon 10 (nonionic surfactant) | 3.0 |
| Diamond Shamrock 2314—VI—26 (polymeric emulsion) | 1.0 |
| Water | 72.3 |

The product was ground in an attrition mill until 50% of the particles were smaller than 1.5 microns.

D. *1.2 × $10^2$ kg/$m^3$ (1 lb/gal) Suspension*

| | |
|---|---|
| Compound of Example 1 | 12.5% |
| Precipitated Silica | 1.0 |
| Antifoam | 0.2 |
| 2% Xanthan Solution | 10.0 |
| Makon 10 (nonionic surfactant) | 4.0 |
| Water | 72.3 |

The product was ground in an attrition mill until 50% of the particles were smaller than 1.5 microns.

E. *1.2 × $10^2$ kg/$m^3$ (1 lb/gal) Suspension*

| | |
|---|---|
| Compound of Example 1 | 12.5% |
| Precipitated Silica | 1.0 |
| Antifoam | 0.2 |
| Makon 10 (nonionic surfactant) | 3.0 |
| 2% Xanthan Solution | 10.0 |
| Polyfon MT—803 (hydrophobic rosin) | 1.0 |
| Water | 72.3 |

The product was ground in an attrition mill until 50% of the particles were smaller than 1.5 microns.

F. *1.2 × 10² kg/m³ (1 lb/gal) Suspension*

| | |
|---|---|
| Compound of Example 2 | 12.5% |
| Precipitated Silica | 1.0 |
| Antifoam | 0.2 |
| 2% Xanthan Solution | 10.0 |
| Makon 10 (nonionic surfactant) | 3.0 |
| Hydrophobic Rosin | 1.0 |
| Water | 72.3 |

The hydrophobic rosin was Polyfon MT—603 in batch F1 and was Polyfon MT—803 in batch F2. The product was ground in an attrition mill until 50% of the particles were smaller than 1.5 microns.

G. *1.2 × 10² kg/m³ (1 lb/gal) Emulsifiable Concentrate*

| | |
|---|---|
| Compound of Example 2 | 11.6% |
| Aromatic Naptha | 84.4 |
| Toximul D (surfactant blend) | 2.0 |
| Sponto AD6—29 (nonionic surfactant) | 2.0 |

H. *1.2 × 10² kg/m³ (1 lb/gal) Emulsifiable Concentrate*

| | |
|---|---|
| Compound of Example 2 | 12.4% |
| Propylene Glycol, Methyl Ether | 12.2 |
| Aromatic Naptha | 65.4 |
| Toximul H (nonionic surfactant) | 9.5 |
| Toximul D (surfactant blend) | 0.5 |

I. *3.6 × 10² kg/m³ (3 lb/gal) Emulsifiable Concentrate*

| | |
|---|---|
| Compound of Example 1 | 37.5% |
| Acetophenone | 57.5 |
| Sponto AD6—29 (nonionic surfactant) | 5.0 |

J. *2.4 × 10² kg/m³ (2 lb/gal) Emulsifiable Concentrate*

| | |
|---|---|
| Compound of Example 1 | 25.0% |
| Acetophenone | 69.5 |
| Sponto AD6—29 (nonionic surfactant | 5.0 |
| Makon 10 (nonionic surfactant) | 0.5 |

K. *2.4 × $10^2$ kg/$m^3$ (2 lb/gal) Emulsifiable Concentrate*

| | |
|---|---|
| Compound of Example 1 | 25.0% |
| Aromatic Naphtha | 70.0 |
| Toximul D (surfactant blend) | 2.5 |
| Sponto AD6—29 (nonionic surfactant) | 2.5 |

L. *1.8 × $10^2$ kg/$m^3$ (1.5 lb/gal) Emulsifiable Concentrate*

| | |
|---|---|
| Compound of Example 2 | 18.75% |
| Aromatic Naphtha | 75.25 |
| Toximul D (surfactant blend) | 3.0 |
| Sponto AD6—29 (nonionic surfactant) | 3.0 |

M. *1.2 × $10^2$ kg/$m^3$ (1 lb/gal) Emulsifiable Concentrate*

| | |
|---|---|
| Compound of Example 1 | 12.2% |
| Propylene Glycol, Methyl Ether | 12.2 |
| Aromatic Naphtha | 65.6 |
| Toximul H (nonionic surfactant) | 10.0 |

N. *1.2 × $10^2$ kg/$m^3$ (1 lb/gal) Emulsifiable Concentrate*

| | |
|---|---|
| Compound of Example 2 | 11.6% |
| Aromatic Naphtha | 85.4 |
| Toximul D (surfactant blend) | 1.5 |
| Sponto AD6—29 (nonionic surfactant) | 1.5 |

O. *1.2 × $10^2$ kg/$m^3$ (1 lb/gal) Suspension*

| | |
|---|---|
| Compound of Example 2 | 12.5% |
| Precipitated Silica | 1.0 |
| Antifoam | 0.2 |
| 2% Xanthan Solution | 10.0 |
| Makon 10 (nonionic surfactant) | 3.0 |
| Water | 72.3 |

Batch 01 contained 1% of additional Makon 10, and batch 02 contained 1% of Diamond Shamrock 2314—IV—26 (polymeric emulsion). The products were ground in an attrition mill until a stable suspension was achieved.

P. *50% Wettable Powder*

| | |
|---|---|
| Compound of Example 1 | 52.1% |
| Sodium Lauryl Sulfate | 5.0 |
| Precipitated Silica | 5.0 |
| Lignin Sulfonate | 5.0 |
| Kaolin | 32.9 |

Q. *2.4 × 10² kg/m³ (2 lb/gal) Emulsifiable Concentrate*

| | |
|---|---|
| Compound of Example 1 | 24.8% |
| Aromatic Naptha | 70.2 |
| Toximul H (nonionic surfactant) | 2.5 |
| Toximul D (surfactant blend) | 2.5 |

R. *1.2 × 10² kg/m³ (1 lb/gal) Emulsifiable Concentrate*

| | |
|---|---|
| Compound of Example 2 | 12.5% |
| Aromatic Naptha | 83.5 |
| Toximul H (nonionic surfactant) | 2.0 |
| Toximul D (surfactant blend) | 2.0 |

S. *1.2 × 10² kg/m³ (1 lb/gal) Suspension*

| | |
|---|---|
| Compound of Example 1 | 12.5% |
| Precipitated Silica | 1.0 |
| 2% Xanthan Solution | 10.0 |
| Antifoam | 0.2 |
| Makon 10 (nonionic surfactant) | 3.0 |
| Polyfac MT803 (hydrophobic rosin) | 1.0 |
| Water | 72.3 |

The product was ground in an attrition mill.

T. *2.4 × 10² kg/m³ (2 lb/gal) Emulsifiable Concentrate*

| | |
|---|---|
| Compound of Example 1 | 22.75% |
| Aromatic Naphtha | 71.25 |
| Sponto AD6—29 (nonionic surfactant) | 2.50 |
| Toximul D (surfactant blend) | 2.50 |
| Petrarch C—09745 (silane surfactant) | 1.00 |

U. *$1.2 \times 10^2$ kg/m³ (1 lb/gal) Emulsifiable Concentrate*

| | |
|---|---|
| Compound of Example 2 | 11.6% |
| Aromatic Naphtha | 83.4 |
| Sponto AD6—29 (nonionic surfactant) | 2.0 |
| Toximul D (surfactant blend) | 2.0 |
| Petrarch C—09745 (silane surfactant) | 1.0 |

V. *$3.6 \times 10^2$ kg/m³ (3 lb/gal) Suspension*

| | |
|---|---|
| Compound of Example 1 | 33.33% |
| Antifoam | 0.15 |
| Makon 10 (nonionic surfactant) | 1.50 |
| 2% Xanthan Solution | 8.00 |
| Preservative | 0.20 |
| Lignin Sulfonate | 0.20 |
| Precipitated Silica | 1.00 |
| Propylene Glycol | 3.50 |
| Water | 52.12 |

The product was ground in an attrition mill.

W. *$2.4 \times 10^2$ kg/m³ (2 lb/gal) Suspension*

| | |
|---|---|
| Compound of Example 2 | 22.00% |
| Antifoam | 0.15 |
| Tergitol 25—L—9 (nonionic surfactant) | 1.50 |
| Propylene Glycol | 3.75 |
| Preservative | 0.20 |
| Veegum (calcium magnesium silicate) | 1.00 |
| Xanthan | 0.20 |
| Diamond Shamrock 2314—VI—26 (polymeric emulsion) | 1.50 |
| Water | 69.70 |

X. *Combination Suspension*

| | |
|---|---|
| Compound of Example 1 | 1.88% |
| Maneb | 7.50 |
| Precipitated Silica | 0.75 |
| Antifoam | 0.15 |
| 2% Xanthan Gum | 7.50 |
| Makon 10 (nonionic surfactant) | 2.25 |
| Polyfon MT—803 (hydrophobic rosin) | 0.75 |
| Water | 79.22 |

The product was ground in an attrition mill until 50% of the particles were smaller than 2 microns by Coulter Counter.

Y. *Combination Suspension*

| | |
|---|---|
| Compound of Example 2 | 1.88% |

The rest of the formula was identical to Composition X.

Z. *Combination Suspension*

| | |
|---|---|
| Compound of Example 2 | 5.4% |
| Makon 10 (nonionic surfactant) | 1.0 |
| Water | 5.6 |
| Dithane FZ (mancozeb 37% suspension) | 88.0 |

The compound, water and Makon were combined and ground in an attrition mill, and the ground suspension was mixed with Dithane.

AA. *Combination Suspension*

| | |
|---|---|
| Compound of Example 1 | 5.4% |

The rest of the formula was identical to that of Composition Z.

**Claims for the Contracting States: BE CH DE FR GB IT LI LU NL SE**

1. A compound of Formula I:

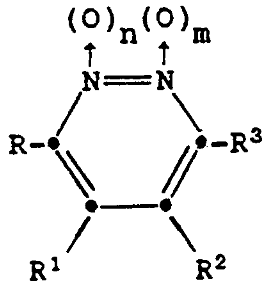

(I)

wherein
$R^3$ is chloro, bromo, methyl, cyano or iodo;

R is chloro, bromo, iodo, methyl, cyano or furan-2-ylmethoxy;
$R^1$ is hydrogen, methyl, ethyl or *n*-propyl;
$R^2$ is

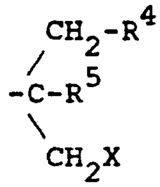

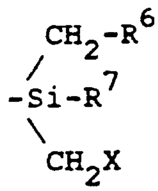

or

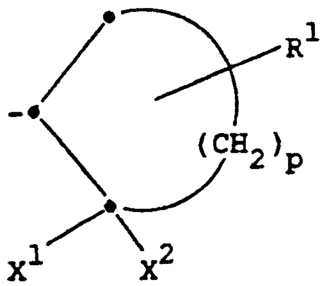

X is fluoro, chloro, bromo or iodo;
$X^1$ and $X^2$ independently represent X or hydrogen, provided that no more than one of $X^1$ and $X^2$ is hydrogen;
$R^4$ is hydrogen, chloro, bromo, methyl or ethyl;
$R^5$ is hydrogen, chloro, methyl, ethyl, chloromethyl or dichloromethyl; or
$R^4$ and $R^5$ combine with the group to which they are attached to form a $C_3$—$C_7$ cycloalkyl group substituted with a $R^1$ group;
$R^6$ is hydrogen, chloro, bromo, methyl or ethyl;
$R^7$ is hydrogen, methyl, ethyl, chloromethyl or dichloromethyl;
one of m and n is 0 or 1, and the other is 0;
p is 0—4.

2. A compound of the formula

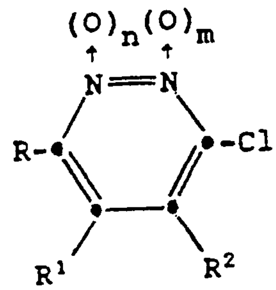

wherein
R is chloro or furan-2-ylmethoxy;
$R^1$ is hydrogen, methyl, ethyl or *n*-propyl;
$R^2$ is

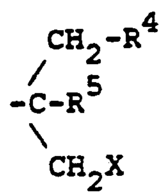

X is fluoro, chloro, bromo or iodo;
$R^4$ is hydrogen, chloro, methyl or ethyl;
$R^5$ is hydrogen, methyl, ethyl, chloromethyl or dichloromethyl;
one of m and n is 0 or 1, and the other is 0.

3. A compound according to Claim 1 or 2 wherein $R^1$ is hydrogen.

4. A compound according to Claims 1 to 3 wherein R is chloro, bromo or methyl.

5 A compound according to Claims 1 to 4 wherein $R^3$ is chloro, bromo or methyl.

6. A compound according to Claims 1 to 5 wherein m and n are 0.

7. A compound of Claim 1 to 6 wherein $R^2$ is

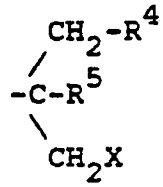

8. A compound according to Claim 7 wherein X is chloro or bromo.

9. 3,6-Dichloro-4-(1-chloromethyl-1-methylethyl)pyridazine.

10. 3,6-Dichloro-4-(1-bromomethyl-1-methylethyl)pyridazine.

11. A method of controlling *Phycomycetous* fungi which comprises applying a compound of Formula I as claimed in any one of claims 1 to 10 to the locus of the fungi.

12. A fungicidal formulation comprising as an active ingredient a compound of Formula I as claimed in any one of claims 1 to 10 associated with a non-phytotoxic carrier therefor.

13. A fungicidal combination composition which comprises a compound of Formula I as claimed in any one of claims 1 to 10, in combination with a dithiocarbamate fungicide of the formula:

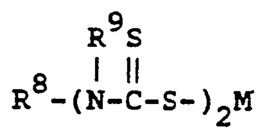

wherein $R^8$ is $C_1$—$C_4$ alkylene;

$R^9$ is $C_1$—$C_3$ alkyl or hydrogen;

M is a divalent metal ion or two monovalent metal ions;

or 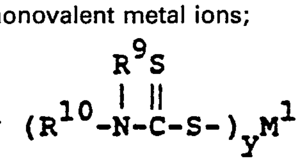

wherein y is 1—3;

$M^1$ is a metal ion of valence 1—3;

$R^{10}$ is $C_1$—$C_4$ alkyl.

14. A process for preparing a compound of Formula I, as claimed in any one of claims 1 to 10, which comprises:

a) halogenating a compound of Formula I, wherein $R^2$ is defined as

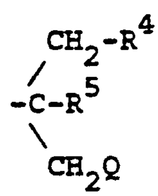 or

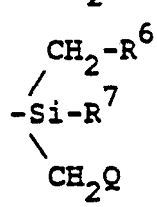 or

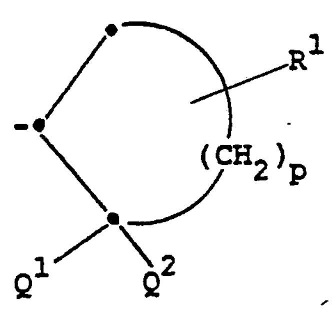

wherein Q or one or both of $Q^1$ and $Q^2$ are hydroxy, hydrogen or a suitable leaving group; or

b) displacement of one or more of Q, $Q^1$ and $Q^2$ halogen atoms with a different halogen;

c) halogenating a compound of Formula I, wherein either one or both of R and $R^3$ are hydroxy,

d) displacing an $R^1$ or $R^3$ halo group, with a different halogen,

e) N-oxidation of a compound of Formula I wherein n and/or m are 0, or

f) displacement of an R or $R^3$ halo group with furan-2-ylmethanol.

**Claims for the Contracting State: AT**

1. A fungicidal formulation comprising as an active ingredient a compound of Formula I:

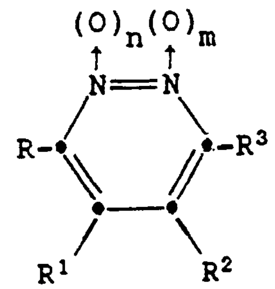

(I)

wherein

$R^3$ is chloro, bromo, methyl, cyano or iodo;

R is chloro, bromo, iodo, methyl, cyano or furan-2-ylmethoxy;

$R^1$ is hydrogen, methyl, ethyl or *n*-propyl;

$R^2$ is

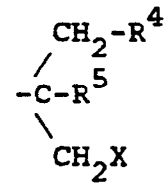

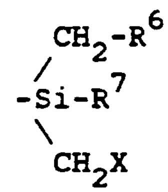

or

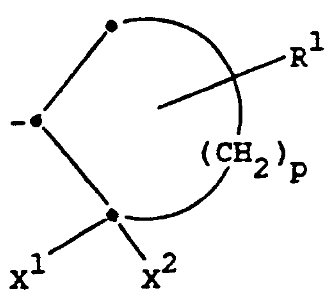

X is fluoro, chloro, bromo or iodo;

$X^1$ and $X^2$ independently represent X or hydrogen, provided that no more than one of $X^1$ and $X^2$ is hydrogen;

$R^4$ is hydrogen, chloro, bromo, methyl or ethyl;

$R^5$ is hydrogen, chloro, methyl, ethyl, chloromethyl or dichloromethyl; or

$R^4$ and $R^5$ combine with the group to which they are attached to form a $C_3$—$C_7$ cycloalkyl group substituted with a $R^1$ group;

$R^6$ is hydrogen, chloro, bromo, methyl or ethyl;

$R^7$ is hydrogen, methyl, ethyl, chloromethyl or dichloromethyl;

one of m and n is 0 or 1, and the other is 0;

p is 0—4; associated with a non-phytotoxic carrier therefor.

2. A fungicidal formulation as claimed in claim 1, wherein the active ingredient is a compound of the formula

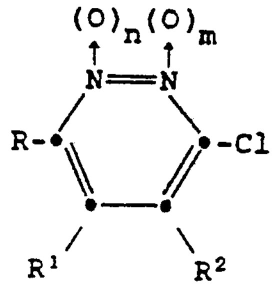

wherein

R is chloro or furan-2-ylmethoxy;

$R^1$ is hydrogen, methyl, ethyl or *n*-propyl;

$R^2$ is

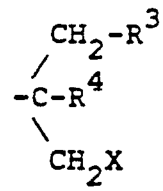

X is fluoro, chloro, bromo or iodo;

$R^3$ is hydrogen, chloro, methyl or ethyl;

$R^4$ is hydrogen, methyl, ethyl, chloromethyl or dichloromethyl;

one of m and n is 0 or 1, and the other is 0.

3. A fungicidal formulation as claimed in claim 1 wherein the active ingredient is a compound of Formula (I) wherein $R^1$ is hydrogen.

4. A fungicidal formulation as claimed in claim 1 wherein the active ingredient is a compound of Formula (I) wherein R is chloro, bromo or methyl.

5. A fungicidal formulation as claimed in claim 1 wherein the active ingredient is a compound of Formula (I) wherein $R^3$ is chloro, bromo or methyl.

6. A fungicidal formulation as claimed in claim 1 wherein the active ingredient is a compound of Formula (I) wherein m and n are 0.

7. A fungicidal formulation as claimed in claim 1 wherein the active ingredient is a compound of Formula (I) wherein $R^2$ is

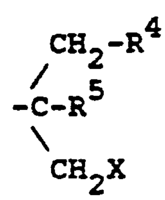

8. A fungicidal formulation as claimed in claim 1 wherein the active ingredient is a compound of Formula (I) wherein X is chloro or bromo.

9. A fungicidal formulation as claimed in claim 1, wherein the active ingredient is 3,6-dichloro-4-(1-chloromethyl-1-methylethyl)pyridazine.

10. A fungicidal formulation as claimed in claim 1, wherein the active ingredient is 3,6-dichloro-4-(1-bromomethyl-1-methylethyl)pyridazine.

11. A method of controlling *Phycomycetous* fungi which comprises applying a compound of Formula I as defined in any one of claims 1 to 10 to the locus of the fungi.

12. A fungicidal composition which comprises a compound of Formula I as defined in any one of claims 1 to 10, in combination with a dithiocarbamate fungicide of the formula:

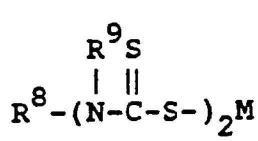

wherein $R^8$ is $C_1$—$C_4$ alkylene;

$R^9$ is $C_1$—$C_3$ alkyl or hydrogen;

M is a divalent metal ion or two monovalent metal ions;

or 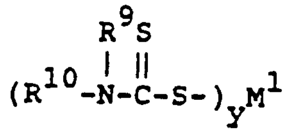

wherein y is 1—3;

$M^1$ is a metal ion of valence 1—3;

$R^{10}$ is $C_1$—$C_4$ alkyl.

13. A process for preparing a compound of Formula I, as defined in any one of claims 1 to 10, which comprises:

a) halogenating a compound of Formula I, wherein $R^2$ is defined as

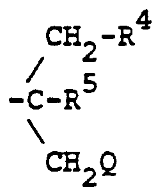 or

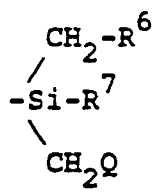 or

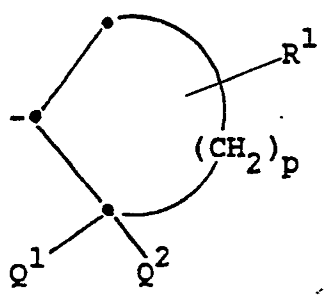

wherein Q or one or both of $Q^1$ and $Q^2$ are hydroxy, hydrogen or a suitable leaving group; or

b) displacement of one or more of Q, $Q^1$ and $Q^2$ halogen atoms with a different halogen;

c) halogenating a compound of Formula I, wherein either one or both of R and $R^3$ are hydroxy,

d) displacing an $R^1$ or $R^3$ halo group, with a different halogen,

e) N-oxidation of a compound of Formula I wherein n and/or m are 0, or

f) displacement of an R or $R^3$ halo group with furan-2-ylmethanol.

**Patentansprüche für die Vertragsstaaten: BE CH DE FR GB IT LI LU NL SE**

1. Verbindung der Formel (I)

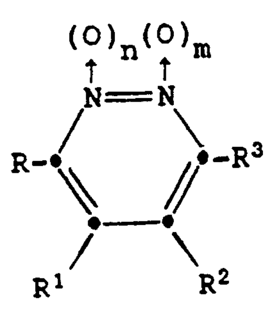 (I)

in der

$R^3$ Chlor, Brom, Methyl, Cyano oder Jod bedeutet;

R Chlor, Brom, Jod, Methyl, Cyano oder Furan-2-ylmethoxy bedeutet;

$R^1$ Wasserstoff, Methyl, Ethyl oder n-Propyl bedeutet;
$R^2$ einen der Reste bedeutet

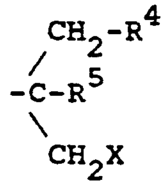 oder

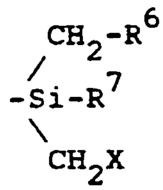 oder

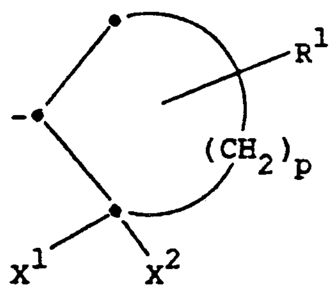

X Fluor, Chlor, Brom oder Jod bedeutet;
$X^1$ und $X^2$ unabhängig voneinander X oder Wasserstoff bedeuten, mit der Maßgabe, daß nicht mehr als einer der Reste $X^1$ und $X^2$ die Bedeutung Wasserstoff hat;
$X^4$ Wasserstoff, Chlor, Brom, Methyl oder Ethyl bedeutet;
$R^5$ Wasserstoff, Chlor, Methyl, Ethyl, Chlormethyl oder Dichlormethyl bedeutet;
oder $R^4$ und $R^5$ zusammen mit der Gruppe, an die sie gebunden sind, einen durch einen $R^1$-Rest substituierten $C_3$—$C_7$-Cycloalkylrest bedeuten;
$R^6$ Wasserstoff, Chlor, Brom, Methyl oder Ethyl bedeutet;
$R^7$ Wasserstoff, Methyl, Ethyl, Chlormethyl oder Dichlormethyl bedeutet;
einer der Indices m und n den Wert 0 oder 1 hat und andere den Wert 0 hat; und
p einen Wert von 0 bis 4 hat.

2. Verbindung der Formel

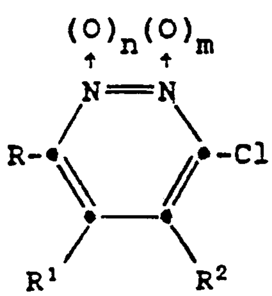

in der
R Chlor oder Furan-2-ylmethoxy bedeutet;
$R^1$ Wasserstoff, Methyl, Ethyl order n-Propyl bedeutet;
$R^2$ die Bedeutung

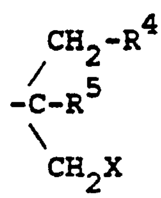

hat;
X Fluor, Chlor, Brom oder Jod bedeutet;
$R^4$ Wasserstoff, Chlor, Methyl oder Ethyl bedeutet;
$R^5$ Wasserstoff, Methyl, Ethyl, Chlormethyl oder Dichlormethyl bedeutet; und
einer der Indices m und n den Wert 0 oder 1 und der andere den wert 0 hat.

3. Verbindung nach Anspruch 1 oder 2, wobei $R^1$ Wasserstoff bedeutet.

4. Verbindung nach Anspruch 1 bis 3, wobei R Chlor, Brom oder Methyl bedeutet.

5. Verbindung nach Anspruch 1 bis 4, wobei $R^3$ Chlor, Brom oder Methyl bedeutet.

6. Verbindung nach Anspruch 1 bis 5, wobei m und n den Wert 0 haben.

7. Verbindung nach Anspruch 1 bis 6, wobei $R^2$ die Bedeutung

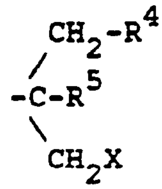

hat:

8. Verbindung nach Anspruch 7, wobei X Chlor oder Brom bedeutet.

9. 3,6-Dichlor-4-(1-chlormethyl-1-methylethyl)-pyridazin.

10. 3,6-Dichlor-4-(1-brommethyl-1-methylethyl)-pyridazin.

11. Verfahren zur Bekämpfung von Phycomycetes-Pilzen, das das Aufbringen einer Verbindung der Formel I nach einem der Ansprüche 1 bis 10 auf die Stelle der Pilze umfaßt.

12. Fungizides Präparat, enthaltend als Wirkstoff eine Verbindung der Formel I nach einem der Ansprüche 1 bis 10 zusammen mit einem nicht-phytotoxischen Träger hierfür.

13. Fungizides Kombinationspräparat, das eine Verbindung der Formel I nach einem der Ansprüche 1 bis 10 in Kombination mit einem Dithiocarbamat-Fungizid der folgenden Formel enthält

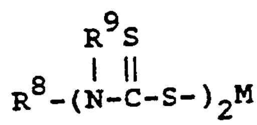

worin $R^8$ $C_1$—$C_4$-Alkylen bedeutet;

$R^9$ $C_1$—$C_3$-Alkyl oder Wasserstoff bedeutet;

M ein zweiwertiges Metallion oder zwei einwertige Metallionen bedeuten; oder

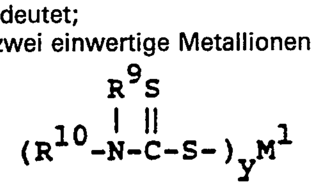

in der

y einen Wert von 1 bis 3 hat;

$M^1$ ein Metallion der Wertigkeit 1 bis 3 bedeutet;

$R^{10}$ $C_1$—$C_4$-Alkyl bedeutet.

14. Verfahren zur Herstellung einer Verbindung der Formel I nach einem der Ansprüche 1 bis 10, umfassend folgende Stufen:

a) Halogenieren einer Verbindung der allgemeinen Formel I, in der $R^2$ definiert ist als

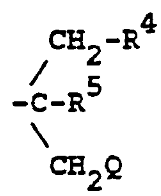

oder

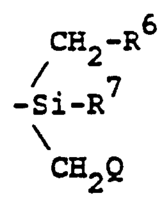

oder

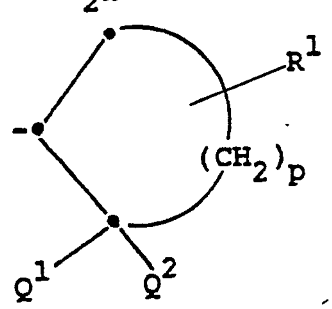

worin Q oder eine oder beide der Reste $Q^1$ und $Q^2$ Hydroxyl, Wasserstoff oder eine geeignete austretende Gruppe bedeuten; oder

b) Ersetzen von einem oder mehreren der Q-, $Q^1$- und $Q^2$-Halogenatome durch ein unterschiedliches Halogen;

c) Halogenieren einer Verbindung der Formel I, in der entweder einer oder beide der Reste R und $R^3$ Hydroxyl bedeuten,

d) Ersetzen einer $R^1$- oder $R^3$-Halogengruppe durch ein unterschiedliches Halogen,

e) N-Oxidation einer Verbindung der Formel I, in der n und/oder m den Wert 0 haben, oder

f) Ersetzen einer R- oder $R^3$-Halogengruppe durch Furan-2-ylmethanol.

**Patentansprüche für den Vertragsstaat: AT**

1. Fungizides Präparat, enthaltend als Wirkstoff eine Verbindung der Formel I

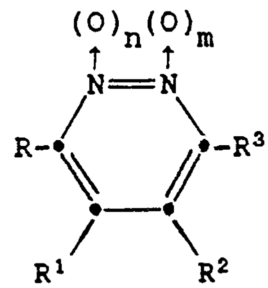

(I)

in der

$R^3$ Chlor, Brom, Methyl, Cyano oder Jod bedeutet;

R Chlor, Brom, Jod, Methyl, Cyano oder Furan-2-ylmethoxy bedeutet;

$R^1$ Wasserstoff, Methyl, Ethyl oder n-Propyl bedeutet;

$R^2$ einen der Reste bedeutet

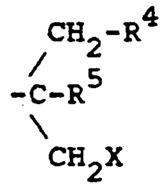

oder

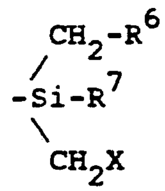

oder

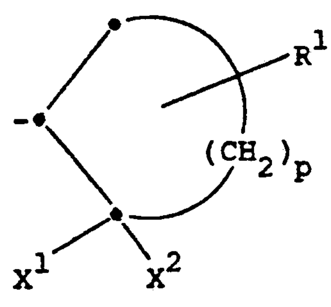

X Fluor, Chlor, Brom oder Jod bedeutet;

$X^1$ und $X^2$ unabhängig voneinander X oder Wasserstoff bedeuten, mit der Maßgabe, daß nicht mehr als einer der Reste $X^1$ und $X^2$ die Bedeutung Wasserstoff hat;

$X^4$ Wasserstoff, Chlor, Brom, Methyl oder Ethyl bedeutet;

$R^5$ Wasserstoff, Chlor, Methyl, Ethyl, Chlormethyl oder Dichlormethyl bedeutet;

oder $R^4$ und $R^5$ zusammen mit der Gruppe, an die sie gebunden sind, einen durch einen $R^1$-Rest substituierten $C_3$—$C_7$-Cycloalkylrest bedeuten;

$R^6$ Wasserstoff, Chlor, Brom, Methyl oder Ethyl bedeutet;
$R^7$ Wasserstoff, Methyl, Ethyl, Chlormethyl oder Dichlormethyl bedeutet;
einer der Indices m und n den Wert 0 oder 1 hat und der andere den Wert 0 hat; und
p einen Wert von 0 bis 4 hat;
zusammen mit einem nicht-phytotoxischen Träger hierfür.

2. Fungizides Präparat nach Anspruch 1, wobei es sich beim Wirkstoff um eine Verbindung der folgenden Formel haldelt

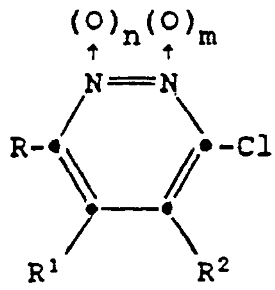

in der
R Chlor oder Furan-2-ylmethoxy bedeutet;
$R^1$ Wasserstoff, Methyl, Ethyl order n-Propyl bedeutet;
$R^2$ die Bedeutung

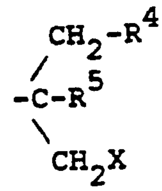

hat;
X Fluor, Chlor, Brom oder Jod bedeutet;
$R^4$ Wasserstoff, Chlor, Methyl oder Ethyl bedeutet;
$R^5$ Wasserstoff, Methyl, Ethyl, Chlormethyl oder Dichlormethyl bedeutet und
einer der Indices m und n den Wert 0 oder 1 und der andere den Wert 0 hat.

3. Fungizides Präparat nach Anspruch 1, wobei es sich beim Wirkstoff um eine Verbindung der Formel (I) handelt, in der $R^1$ Wasserstoff bedeutet.

4. Fungizides Präparat nach Anspruch 1, wobei es sich beim Wirkstoff um eine Verbindung der Formel (I) handelt, in der R Chlor, Brom oder Methyl bedeutet.

5. Fungizides Präparat nach Anspruch 1, wobei es sich beim Wirkstoff um eine Verbindung der Formel (I) handelt, in der $R^3$ Chlor, Brom oder Methyl bedeutet.

6. Fungizides Präparat nach Anspruch 1, wobei es sich beim Wirkstoff um eine Verbindung der Formel (I) handelt, in der m und n den Wert 0 haben.

7. Fungizides Präparat nach Anspruch 1, wobei es sich beim Wirkstoff um eine Verbindung der Formel (I) handelt, in der $R^2$ die Bedeutung

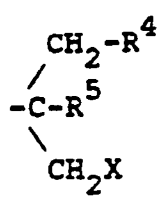

hat.

8. Fungizides Präparat nach Anspruch 1, wobei es sich beim Wirkstoff um eine Verbindung der Formel (I) handelt, in der X Chlor oder Brom bedeutet.

9. Fungizides Präparat nach Anspruch 1, wobei es sich beim Wirkstoff um 3,6-Dichlor-4-(1-chlormethyl-1-methylethyl)-pyridazin handelt.

10. Fungizides Präparat nach Anspruch 1, wobei es sich beim Wirkstoff um 3,6-Dichlor-4-(1-brommethyl-1-methylethyl)-pyridazin handelt.

11. Verfahren zur Bekämpfung von Phycomycetes-Pilzen, das das Aufbringen einer Verbindung der Formel I gemäß einem der Ansprüche 1 bis 10 auf die Stelle der Pilze umfaßt.

12. Fungizide Zusammensetzung, enthaltend eine Verbindung der Formel I nach einem der Ansprüche 1 bis 10 in Kombination mit einem Dithiocarbamat-Fugizid der Formel

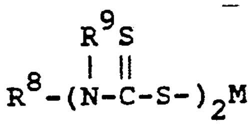

worin $R^8$ $C_1$—$C_4$-Alkylen bedeutet;
$R^9$ $C_1$—$C_3$-Alkyl oder Wasserstoff bedeutet;
M ein zweiwertiges Metallion oder zwei einwertige Metallionen bedeuten; oder

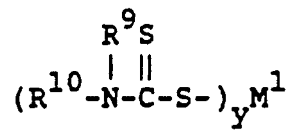

in der
y einen Wert von 1 bis 3 hat;
$M^1$ ein Metallion der Wertigkeit 1 bis 3 bedeutet;
$R^{10}$ $C_1$—$C_4$-Alkyl bedeutet.

13. Verfahren zur Herstellung einer Verbindung der Formel I nach einem der Ansprüche 1 bis 10, umfassend folgende Stufen:

a) Halogenieren einer Verbindung der allgemeinen Formel I, in der $R^2$ definiert ist als

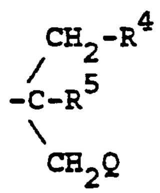

oder

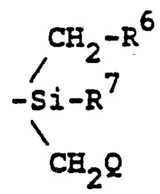

oder

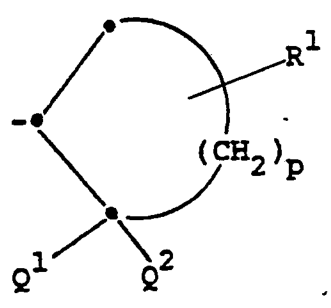

worin Q oder eine oder beide der Reste $Q^1$ und $Q^2$ Hydroxyl, Wasserstoff oder eine geeignete austretende Gruppe bedeuten; oder

b) Ersetzen von einem oder mehreren der Q-, $Q^1$- und $Q^2$-Halogenatome durch ein unterschiedliches Halogen;

c) Halogenieren einer Verbindung der Formel I, in der entweder einer oder beide der Reste R und $R^3$ Hydroxyl bedeuten,

d) Ersetzen einer $R^1$- oder $R^3$-Halogengruppe durch ein unterschiedliches Halogen,

e) N-Oxidation einer Verbindung der Formel I, in der n und/oder m den Wert 0 haben, oder

f) Ersetzen einer R- oder $R^3$-Halogengruppe durch Furan-2-ylmethanol.

**Revendications pour les Etats contractants: BE CH DE FR GB IT LI LU NL SE**

1. Composé de formule I:

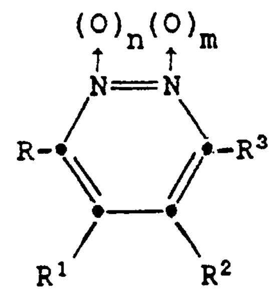

(I)

dans laquelle

$R^3$ représente un atome de chlore, un atome de brome, un groupe méthyle, un groupe cyano ou un atome d'iode;

R représente un atome de chlore, un atome de brome, un atome d'iode, un groupe méthyle, un groupe cyano ou un groupe furan-2-ylméthoxy;

$R^1$ représente un atome d'hydrogène, un groupe méthyle, un groupe éthyle ou un groupe n-propyle;

$R^2$ représente

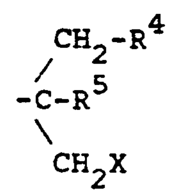

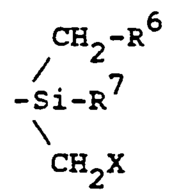

ou

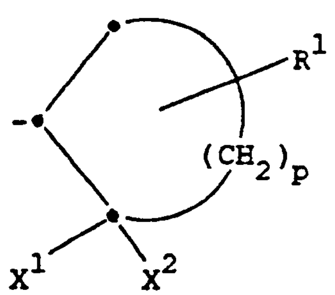

X représente un atome de fluor, un atome de chlore, un atome de brome ou un atome d'iode;

$X^1$ et $X^2$ représentent chacun, indépendamment l'un de l'autre, X ou un atome d'hydrogène, avec cette réserve que pas plus d'un des radicaux $X^1$ et $X^2$ représente un atome d'hydrogène;

$R^4$ représente un atome d'hydrogène, un atome de chlore, un atome de brome, un groupe méthyle ou un groupe éthyle;

$R^5$ représente un atome d'hydrogène, un atome de chlore, un groupe méthyle, un groupe éthyle, un groupe chlorométhyle ou un groupe dichlorométhyle;

ou $R^4$ et $R^5$ se combinent avec le groupe auquel ils sont fixés pour former un groupe cycloalkyle en $C_3$—$C_7$ substitué par un groupe $R^1$;

$R^6$ représente un atome d'hydrogène, un atome de chlore, un atome de brome, un groupe méthyle ou un groupe éthyle;

$R^7$ représente un atome d'hydrogène, un groupe méthyle, un groupe éthyle, un groupe chlorométhyle ou un groupe dichlorométhyle;

une des lettres m et n représente O ou 1, et l'autre représente 0;

p est égal à 0—4.

2. Composé de formule

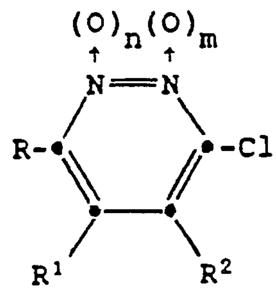

dans laquelle

R représente un atome de chlore ou un groupe furan-2-ylméthoxy;

$R^1$ représente un atome d'hydrogène, un groupe méthyle, un groupe éthyle ou un groupe n-propyle;

$R^2$ représente

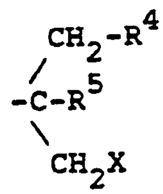

X représente un atome de fluor, un atome de chlore, un atome de brome ou un atome d'iode;

$R^4$ représente un atome d'hydrogène, un atome de chlore, un groupe méthyle ou un groupe éthyle;

$R^5$ représente un atome d'hydrogène, un groupe méthyle, un groupe éthyle, un groupe chlorométhyle ou un groupe dichlorométhyle;

une des lettres m et n est égale à 0 ou 1, et l'autre est égale à 0.

3. Composé selon la revendication 1 ou 2 dans lequel $R^1$ représente un atome d'hydrogène.

4. Composé selon les revendications 1 à 3 dans lequel R représente un atome de chlore, un atome de brome ou un groupe méthyle.

5. Composé selon les revendications 1 à 4 dans lequel $R^3$ représente un atome de chlore, un atome de brome ou un groupe méthyle.

6. Composé selon les revendications 1 à 5 dans lequel m et n sont égaux à 0.

7. Composé selon les revendications 1 à 6 dans lequel $R^2$ représente

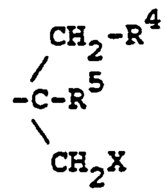

8. Composé selon la revendication 7 dans lequel X représente un atome de chlore ou un atome de brome.

9. 3,6-dichloro-4-(1-chlorométhyl-1-méthyléthyl)pyridazine.

10. 3,6-dichloro-4-(1-bromométhyl-1-méthyléthyl)pyridazine.

11. Procédé en vue de combattre les champigons Phycomycètes consistant à appliquer un composé de formule I selon l'une quelconque des revendications de 1 à 10, au siège des champignons.

12. Formulation fongicide comprenant comme ingrédient actif un composé de formule I selon l'une quelconque des revendications 1 à 10, en association avec un support non phytotoxique, pour ce composé.

13. Combinaison fongicide comprenant un composé de formule I selon l'une quelconque des revendications 1 à 10, en combinaison avec un fonigcide dithiocarbamate de formule:

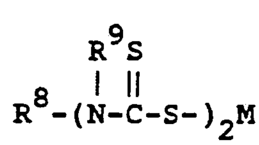

où $R^8$ représente un groupe alkylène en $C_1$—$C_4$;

$R^9$ représente un groupe alkyle en $C_1$—$C_3$ ou un atome d'hydrogène;

M représente un ion métallique divalent ou deux ions métalliques monovalents;

ou 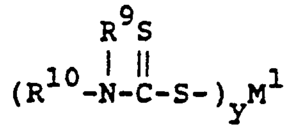

où y représente 1—3;

$M^1$ représente un ion métallique de valence 1—3;

$R^{10}$ représente un groupe alkyle en $C_1$—$C_4$.

14. Procédé de préparation d'un composé de formule I selon l'une quelconque des revendications 1 à 10, ce procédé consistant à:

a) halogéner un composé de formule (I) dans laquelle $R^2$ est défini comme étant:

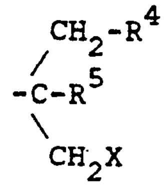

ou

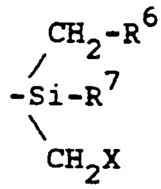

ou

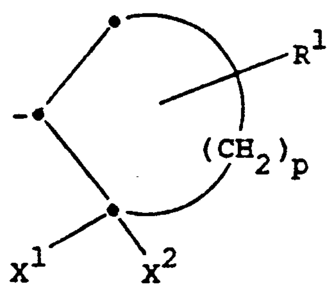

où Q ou un des radicaux $Q^1$ et $Q^2$ ou les deux ensemble représente(nt) un groupe hydroxyle, un atome d'hydrogène ou un groupe approprié qui se sépare; ou

b) remplacer un ou plusieurs atomes d'halogènes de Q, $Q^1$ et $Q^2$ par un atome d'halogène différent;

c) halogéner un composé de formule I dans laquelle un des radicaux R et $R^3$ ou les deux ensemble représente(nt) un groupe hydroxyle;

d) remplacer un atome d'halogène de $R^1$ ou $R^3$ par un atome d'halogène différent;

e) procéder à la N-oxydation d'un composé de formule I dans laquelle n et/ou m représente(nt) 0, ou

f) remplacer un atome d'halogène de R ou $R^3$ par un groupe furan-2-ylméthanol.

**Revendications pour l'Etat contractant: AT**

1. Formulation fongicide comprenant comme ingrédient actif un composé de formule I:

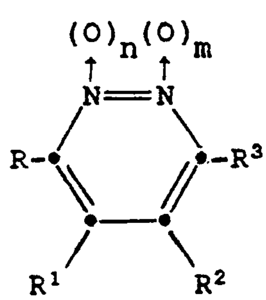

(I)

dans laquelle

$R^3$ représente un atome de chlore, un atome de brome, un groupe méthyle, un groupe cyano ou un atome d'iode;

R représente un atome de chlore, un atome de brome, un atome d'iode, un groupe méthyle, un groupe cyano ou un groupe furan-2-ylméthoxy;

$R^1$ représente un atome d'hydrogène, un groupe méthyle, un groupe éthyle ou un groupe n-propyle;

$R^2$ représente

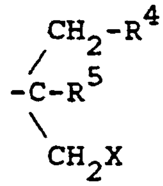

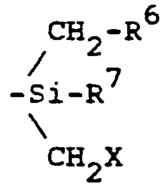

ou

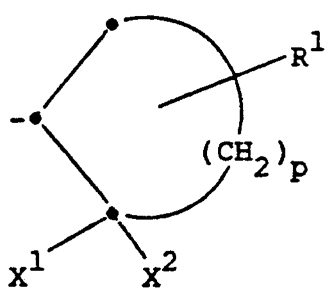

X représente un atome de fluor, un atome de chlore, un atome de brome ou un atome d'iode;

$X^1$ et $X^2$ représentent chacun, indépendamment l'un de l'autre, X ou un atome d'hydrogène, avec cette réserve que pas plus d'un des radicaux $X^1$ et $X^2$ représente un atome d'hydrogène;

$R^4$ représente un atome d'hydrogène, un atome de chlore, un atome de brome, un groupe méthyle ou un groupe éthyle;

$R^5$ représente un atome d'hydrogène, un atome de chlore, un groupe méthyle, un groupe éthyle, un groupe chlorométhyle ou un groupe dichlorométhyle;

ou $R^4$ et $R^5$ se combinent avec le groupe auquel ils sont fixés pour former un groupe cycloalkyle en $C_3$—$C_7$ substitué par un groupe $R^1$;

$R^6$ représente un atome d'hydrogène, un atome de chlore, un atome de brome, un groupe méthyle ou un groupe éthyle;

$R^7$ représente un atome d'hydrogène, un groupe méthyle, un groupe éthyle, un groupe chlorométhyle ou un groupe dichlorométhyle;

une des lettres m et n représente 0 ou 1, et l'autre représente 0;

p est égal à 0—4.

2. Formulation fongicide selon la revendication 1, dans laquelle l'ingrédient actif est un composé de formule

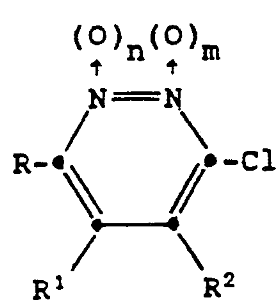

dans laquelle

R représente un atome de chlore ou un groupe furan-2-ylméthoxy;

$R^1$ représente un atome d'hydrogène, un groupe méthyle, un groupe éthyle ou un groupe n-propyle;
$R^2$ représente

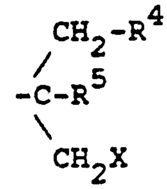

X représente un atome de fluor, un atome de chlore, un atome de brome ou un atome d'iode;
$R^4$ représente un atome d'hydrogène, un atome de chlore, un groupe méthyle ou un groupe éthyle;
$R^5$ représente un atome d'hydrogène, un groupe méthyle, un groupe éthyle, un groupe chlorométhyle ou un groupe dichlorométhyle;
une des lettres m et n est égale à 0 ou 1, et l'autre est égale à 0.

3. Formulation fongicide selon la revendication 1, dans laquelle l'ingrédient actif est un composé de formule (I) dans lequelle $R^1$ représente un atome d'hydrogène.

4. Formulation fongicide selon la revendication 1, dans laquelle l'ingrédient actif est un composé de formule (I) dans lequelle R représente un atome de chlore, un atome de brome ou un groupe méthyle.

5. Formulation fongicide selon la revendication 1, dans laquelle l'ingrédient actif est un composé de formule (I) dans lequelle $R^3$ représente un atome de chlore, un atome de brome ou un groupe méthyle.

6. Formulation fongicide selon la revendication 1, dans laquelle l'ingrédient actif est un composé de formule (I) dans lequelle m et n sont égaux à 0.

7. Formulation fongicide selon la revendication 1, dans laquelle l'ingrédient actif est un composé de formule (I) dans laquelle $R^2$ représente

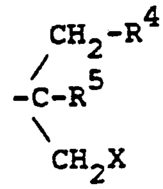

8. Formulation fongicide selon la revendication 1, dans laquelle l'ingrédient actif est un composé de formule (I) dans lequelle X représente un atome de chlore ou un atome de brome.

9. Formulation fongicide selon la revendication 1, dans laquelle l'ingrédient actif est la 3,6-dichloro-4-(1-chlorométhyl-1-méthyléthyl)pyridazine.

10. Formulation fongicide selon la revendication 1, dans laquelle l'ingrédient actif est la 3,6-dichloro-4-(1-bromométhyl-1-méthyléthyl)pyridazine.

11. Procédé en vue de combattre les champigons Phycomycètes consistant à appliquer un composé de formule I selon l'une quelconque des revendications de 1 à 10, au siège des champignons.

12. Combinaison fongicide comprenant un composé de formule I selon l'une quelconque des revendications 1 à 10, en combinaison avec un fongicide dithiocarbamate de formule:

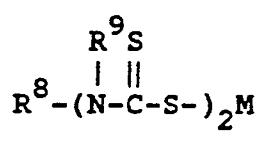

où $R^8$ représente un groupe alkylène en $C_1$—$C_4$;
$R^9$ représente un groupe alkyle en $C_1$—$C_3$ ou un atome d'hydrogène;
M représente un ion métallique divalent ou deux ions métalliques monovalents;

ou 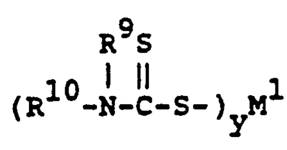

où y représente 1—3;
$M^1$ représente un ion métallique de valence 1—3;
$R^{10}$ représente un groupe alkyle en $C_1$—$C_4$.

13. Procédé de préparation d'un composé de formule I selon l'une quelconque des revendications 1 à 10, ce procédé consistant à:

a) halogéner un composé de formule I dans laquelle $R^2$ est défini comme étant:

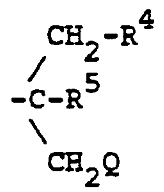

ou

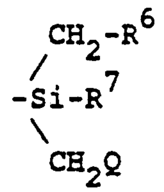

ou

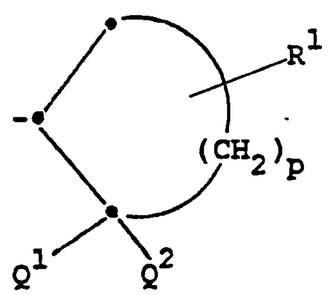

où Q ou un des radicaux $Q^1$ et $Q^2$ ou les deux ensemble représente(nt) un groupe hydroxyle, un atome d'hydrogène ou un groupe approprié qui se sépare; ou

b) remplacer un ou plusieurs atomes d'halogènes de Q, $Q^1$ et $Q^2$ par un atome d'halogène différent;

c) halogéner un composé de formule I dans laquelle un des radicaux R et $R^3$ ou les deux ensemble représente(nt) un groupe hydroxyle;

d) remplacer un atome d'halogène de $R^1$ ou $R^3$ par un atome d'halogène différent;

e) procéder à la N-oxydation d'un composé de formule I dans laquelle n et/ou m représente(nt) 0, ou

f) remplacer un atome d'halogène de R ou $R^3$ par un groupe furan-2-ylméthanol.